# EUROPEAN PATENT SPECIFICATION

(11) **EP 3 931 178 B1**
(45) Date of publication and mention of the grant of the patent: **25.06.2025**
(21) Application number: 20767071.2
(22) Date of filing: 02.03.2020
(51) Int. Cl.: A61K 31/15, A61P 25/28, C07C 243/24, C07C 243/36, C08K 5/25

(54) **COMPOUNDS FOR PREVENTION AND TREATMENT OF POST-OPERATIVE COGNITIVE DYSFUNCTION**
VERBINDUNGEN ZUR VORBEUGUNG UND BEHANDLUNG VON POSTOPERATIVER KOGNITIVER DYSFUNKTION
COMPOSÉS POUR LA PRÉVENTION ET LE TRAITEMENT D'UN DYSFONCTIONNEMENT COGNITIF POST-OPÉRATOIRE

(30) Priority: 01.03.2019 US 201962812309 P; 01.11.2019 US 201962929694 P; 19.11.2019 US 201962937653 P
(43) Date of publication of application: 05.01.2022
(73) Proprietor: Abrexa Pharmaceuticals, Inc., San Diego, CA 92103 (US)
(72) Inventor: VILLAFRANCA, Jesus E., San Diego, CA 92103 (US); KISSINGER, Charles Richard, San Diego, CA 92103 (US)
(74) Representative: Cohausz & Florack
(86) International application number: PCT/US2020/020709
(87) International publication number: WO 2020/180823

(56) References cited:
- WO-A1-2009/052116
- WO-A2-2017/140684
- US-A- 4 017 540
- US-A1- 2007 238 700
- US-A1- 2010 004 302
- US-A1- 2010 099 775
- US-A1- 2010 305 181
- US-A1- 2017 174 614
- QI CHEN ET AL: "A Novel Neurotrophic Drug for Cognitive Enhancement and Alzheimer's Disease", PLOS ONE, vol. 6, no. 12, 14 December 2011 (2011-12-14), pages e27865, XP055134062, ISSN: 1932-6203, DOI: 10.1371/journal.pone.0027865
- MARGUERITE PRIOR ET AL: "The neurotrophic compound J147 reverses cognitive impairment in aged Alzheimer's disease mice", ALZHEIMERS RES THER, BIOMED CENTRAL LTD, LONDON, UK, vol. 5, no. 25, 14 May 2013 (2013-05-14), pages 1 - 19, XP021151849, ISSN: 1758-9193, DOI: 10.1186/ALZRT179
- DATABASE PUBCHEM [online] 24 October 2006 (2006-10-24), Database accession no. 9679644

## Description

### FIELD OF THE INVENTION

The present invention is defined in the appended claims. It relates to the use of certain compounds for the treatment and/or prevention of post-operative cognitive dysfunction. Embodiments not falling within the scope of the appended claims do not form part of the invention. Any references in the description to methods of treatment refer to compounds, pharmaceutical compositions or medicaments of the present invention for use in a method of treatment of the human (or animal) body by therapy.

### BACKGROUND

Post-operative cognitive dysfunction (POCD) is an impairment or decline in cognitive function observed in a subject after a medical procedure or surgery has been conducted. The occurrence of POCD is most often observed after cardiac surgery. However, several recent studies have verified that POCD occurs after non-cardiac surgeries. POCD is common in adult patients of all ages following a major surgery. However, the risk of developing POCD increases with age. The severity and duration of POCD also appears to increase with age, and elderly subjects (e.g., aged 60 years or older) are at high risk of developing long-term cognitive problems following a major surgery. Although the risk of POCD increases with age, the type of surgery conducted may also be relevant. The risk of, or severity of POCD does not appear to correlate with the type of anesthesia. For example. POCD is just as likely to occur after operations that utilize local/regional or general anesthesia. The risk, duration and severity of POCD also appears to correlate with a history of high alcohol intake, a history of smoking, patients with lower educational level, patients with a prior history of stroke, and patients with pre-existing cognitive disorders (e.g., mild cognitive impairment).

The etiology of POCD and underlying biological mechanisms that cause POCD are not well understood. WO 2017/140684 discloses APJ receptor agonists for use in the treatment of POCD. US 2010/099775 teaches about ubiquinone for use in the treatment of POCD.

### SUMMARY

Provided a compound for use in a method of preventing, reducing, delaying or treating post-operative cognitive dysfunction (POCD), or one or more symptoms thereof, in a subject comprising administering to the subject a therapeutically effective amount of a compound disclosed herein (e.g., sometimes referred to a "compound of the invention"). The compound of the invention comprises the structure of Formula IV; 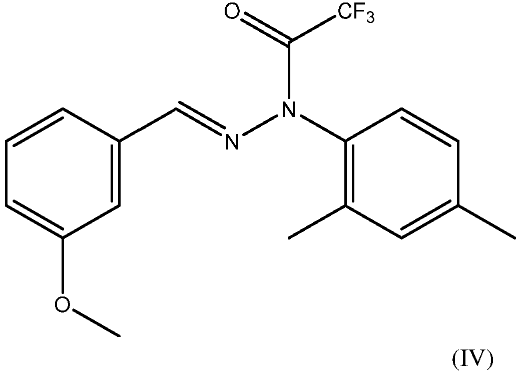 or a pharmaceutically acceptable salt, stereoisomer or tautomer thereof.

According to the invention, POCD is induced by, or is a result of a medical procedure. In some embodiments, the medical procedure comprises major surgery. In certain embodiments the major surgery comprises a surgical procedure having a duration of 0.5 to 20 hours. In some embodiments, the medical procedure is selected from cardiac surgery, angioplasty, organ transplant surgery, complete or partial removal of an organ or tissue, brain surgery, bone replacement or repair surgery, abdominal surgery, facial reconstruction or repair surgery, and pelvic floor surgery.

In some embodiments, the subject is human. In some embodiments, the subject is over the age of 60, or over the age of 65. In some embodiments, the subject displays stable cognitive function prior to the medical procedure. In some embodiments, the subject was not previously diagnosed with a cognitive disorder or neurodegenerative disease prior to the medical procedure.

In certain embodiments, the subject is diagnosed with a neurodegenerative disease prior to the medical procedure. In some embodiments, the neurodegenerative disease is selected from Alzheimer's disease, Parkinson's disease, Huntington's disease, amyotrophic lateral sclerosis, glaucoma, retinal degeneration, macular degeneration, age-related hearing loss, mild cognitive impairment, dementia, progressive supranuclear palsy, spinocerebellar ataxia, retinal neuropathy, peripheral neuropathy, diabetic neuropathy, background neuropathy, familial amyloid polyneuropathy, systemic senile amyloidosis, prion disease, scrapic, bovine spongiform encephalopathy, Creutzfeldt-Jakob disease, Gerstmann-Straussler-Scheinker syndrome and amyloidosis.

In certain aspects, a compound of the invention is administered prior to, perioperatively, during or after the medical procedure or surgery. In some embodiments, the compound of the invention is administered at a dose of 0.5 mg/kg to 100 mg/kg. In some embodiments, the compound of the invention is administered orally or intravenously.

### DETAILED DESCRIPTION OF THE INVENTION

Presented herein are compounds for treating and/or preventing POCD.

### Compounds of the disclosure

In some aspects of the disclosure provided herein is a compound for use in preventing or treating POCD, or one or more symptoms thereof. In some aspects of the disclosure a compound of the invention comprises the structure of Formula I; 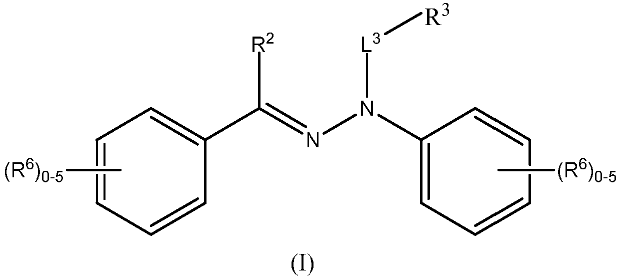 or a pharmaceutically acceptable salt, stereoisomer or tautomer thereof. In some embodiments of Formula I, R² is hydrogen (H) or methyl; R³ is a methyl, a fluorine substituted alkyl *(e.g.,* fluoromethyl, difluoromethyl, or trifluoromethyl), or a bromine substituted alkyl *(e.g.,* bromomethyl, dibromomethyl, tribromomethyl); L³ is a carbonyl; and R⁶ at each occurrence is independently selected from alkyl, substituted alkyl, alkenyl, substituted alkenyl, cycloalkyl, substituted cycloalkyl, hydroxyl, methoxy, alkoxy, substituted alkoxy, aryloxy, substituted aryloxy, mercapto, alkylthio, arylthio, carbonyl, carboxyl, aryl, substituted aryl, substituted heterocyclic, halogen, cyano, cyanoalkyl, amine, methyl amine, dimethyl amine, nitro, amino, amidino, carbamate, CF₃, OCF₃, S(O)ₙR⁷, and C(O)R⁸, or two R⁶ at adjacent positions combine to form an optionally substituted heteroaryl or heteroalkyl ring fused with the adjoining phenyl moiety; where R⁷ is selected from H, R⁹, NH₂, HNR⁹ and NR⁹R¹⁰; R⁸ is selected from OH, OR⁹, NH₂, NHR⁹ and NR⁹R¹⁰; where R⁹ and R¹⁰ at each occurrence are independently an optionally substituted alkyl; and n is 1 or 2.

In certain aspects of the disclosure of Formula I, R⁶ at each occurrence is independently selected from alkyl, substituted alkyl, alkenyl, substituted alkenyl, hydroxyl, alkoxy, methoxy, substituted alkoxy, halogen, carbonyl, carboxyl, or C(O)R⁸; and in certain such aspects, R⁶ at each occurrence is methyl, methoxy, perfluoromethyl, perfluoromethoxy, hydroxyl, Cl, F, or I. In some aspects of the disclosure of Formula I, L³ is carbonyl, R³ is CF₃, R² is H, and R⁶ is null or H at every occurrence. In some aspects of the disclosure of Formula I, L³ is carbonyl, R³ is CF₃, R² is H, and R⁶ is independently selected from methyl or methoxy, at each occurrence. In some aspects of the disclosure of Formula I, L³ is carbonyl, R³ is CF₃, R² is methyl, and R⁶ is independently selected from methyl or methoxy, at each occurrence.

The disclosure further relates to a compound that comprises the structure of Formula II; 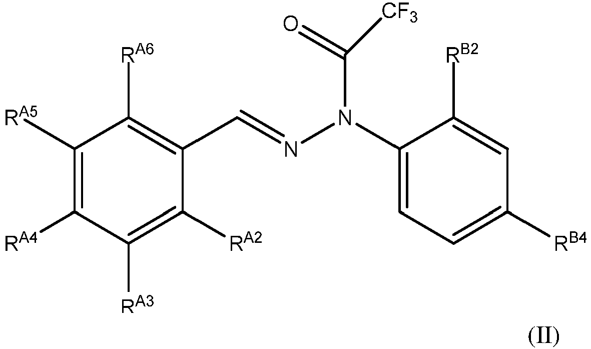 or a pharmaceutically acceptable salt, stereoisomer or tautomer thereof, where:
(i) R^{A2}, R^{A4}, R^{A5}, and R^{A6} is H, R^{A3} is methoxy, R^{B2} is methyl, and R^{B4} is methyl;
(ii) R^{A2}, R^{A3}, R^{A5}, and R^{A6} is H, R^{A4} is methoxy, R^{B2} is methyl, and R^{B4} is methyl;
(iii) R^{A2}, R^{A3}, R^{A4}, R^{A5}, and R^{A6} is H, R^{B2} is H, and R^{B4} is H;
(iv) R^{A2}, R^{A3}, R^{A4}, R^{A5}, and R^{A6} is H, R^{B2} is methyl, and R^{B4} is methyl;
(v) R^{A2}, R^{A4}, R^{A5}, and R^{A6} is H, R^{A3} is methoxy, R^{B2} is H, and R^{B4} is H;
(vi) R^{A2}, R^{A3}, R^{A4}, R^{A5}, and R^{A6} is H, R^{B2} is H, and R^{B4} is methyl;
(vii) R^{A2}, R^{A4}, R^{A5}, and R^{A6} is H, R^{A3} is methoxy, R^{B2} is H, and R^{B4} is methyl;
(viii) R^{A2}, R^{A3}, R^{A4}, R^{A5}, and R^{A6} is H, R^{B2} is methyl, and R^{B4} is H;
(ix) R^{A2}, R^{A4}, R^{A5}, and R^{A6} is H, R^{A3} is methoxy, R^{B2} is methyl, and R^{B4} is H;
(x) R^{A2}, R^{A3}, R^{A5}, and R^{A6} is H, R^{A4} is COOH, R^{B2} is methyl, and R^{B4} is methyl;
(xi) R^{A2}, R^{A4}, and R^{A5} is H, R^{A3} and R^{A6} is hydroxyl, R^{B2} is methyl, and R^{B4} is methyl;
(xii) R^{A2}, R^{A4}, and R^{A6} is H, R^{A3} and R^{A5} is hydroxyl, R^{B2} is methyl, and R^{B4} is methyl;
(xiii) R^{A2}, R^{A4}, and R^{A5} is H, R^{A3} is methoxy, R^{A6} is F, R^{B2} is H, and R^{B4} is Cl;
(xiv) R^{A3} and R^{A5}is H, R^{A2} and R^{A6} is F, R^{A4} is hydroxyl, R^{A6} is F, R^{B2} is H, and R^{B4} is F;
(xv) R^{A2}, R^{A4}, and R^{A6} is H, R^{A3} is hydroxyl, R^{A5} is F, R^{B2} is H, and R^{B4} is F; or
(xvi) R^{A2}, R^{A5}, and R^{A6} is H, R^{A3} and R^{A4} taken together are -O-CH₂-O-, R^{A5} is F, R^{B2} is H, and R^{B4} is F.

In some aspects of the disclosure of the compound of Formula II, R^{A2}, R^{A5}, and R^{A6} are H, R^{A3} is methoxy, R^{B2} and R^{B4} are methyl, and R^{A4} is selected from H, NO₂, OH, methoxy, phenol, methyl, Fluorine (F), N(CH₃)₂, CHC(CN)₂ and O-tert-butyldimethylsilyl (OTBDMS). In some aspects of the disclosure of the compound of Formula II, R^{A2}, R^{A4}, R^{A5}, and R^{A6} are H, R^{A3} is methoxy, R^{B2} is methyl, and R^{B4} is methyl. In some aspects of the disclosure of the compound of Formula II, R^{A2}, R^{A3} R^{A5}, and R^{A6} are H, R^{A4} is methoxy, R^{B2} is methyl, and R^{B4} is methyl. In some aspects of the disclosure of the compound of Formula II, R^{A2}, R^{A3}, R^{A4}, R^{A5} and R^{A6} are H, R^{B2} is methyl, and R^{B4} is methyl. In some aspects of the disclosure of the compound of Formula II, R^{A2}, R^{A4}, R^{A5} and R^{A6} are H, R^{A3} is methoxy, R^{B2} is H, and R^{B4} is H. In some aspects of the disclosure of the compound of Formula II, R^{A2}, R^{A3}, R^{A4}, R^{A5} and R^{A6} are H, R^{B2} is H, and R^{B4} is methyl. In some aspects of the disclosure of the compound of Formula II, R^{A2}, R^{A3}, R^{A4}, R^{A5} and R^{A6} are H, R^{B2} is H, and R^{B4} is methyl. In some aspects of the disclosure of the compound of Formula II, R^{A2}, R^{A4}, R^{A5} and R^{A6} are H, R^{A3} is methoxy, R^{B2} is H, and R^{B4} is methyl. In some aspects of the disclosure of the compound of Formula II, R^{A2}, R^{A4}, R^{A5} and R^{A6} are H, R^{A3} is methoxy, R^{B2} is methyl, and R^{B4} is H. In some aspects of the disclosure of the compound of Formula II, R^{A2}, R^{A3}, R^{A4}, R^{A5} and R^{A6} are H, R^{B2} is methyl, and R^{B4} is H. In some aspects of the disclosure of the compound of Formula II, R^{A2}, R^{A3}, R^{A5} and R^{A6} are H, R^{A4} is a carboxyl, R^{B2} is methyl, and R^{B4} is methyl. In some aspects of the disclosure of the compound of Formula II, R^{A2}, R^{A4}, R^{A5} and R^{A6} are H, R^{A3} is a carboxyl, R^{B2} is methyl, and R^{B4} is methyl.

In some aspects of the disclosure, a compound of the invention comprises the structure of Formula VI; 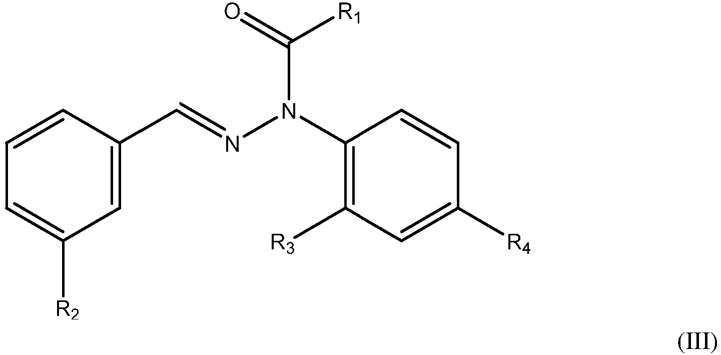 or a pharmaceutically acceptable salt, stereoisomer or tautomer thereof, where R₁ is methyl, fluoromethyl, difluoromethyl, trifluoromethyl, bromomethyl, dibromomethyl or tribromomethyl; R₂ is methyl, methoxy, hydroxyl, halogen, CF₃, OCH₃, OCF₃ or OCBr₃; and R₃ and R₄ are independently selected from hydrogen, hydroxyl, a halogen *(e.g.,* Cl, For Br), methyl, a methoxy, and an amine. In some aspects of the disclosure of Formula III, R₁ is CF₃ (trifluoromethyl), R₂ is OCH₃, and R₃ and R₄ are methyl. In some aspects of the disclosure of Formula III, R₁ is CF₃ (trifluoromethyl), R₂ is OCF₃, and R₃ and R₄ are methyl

The compound of the invention comprises the structure of Formula IV below, or a pharmaceutically acceptable salt, stereoisomer or tautomer thereof.

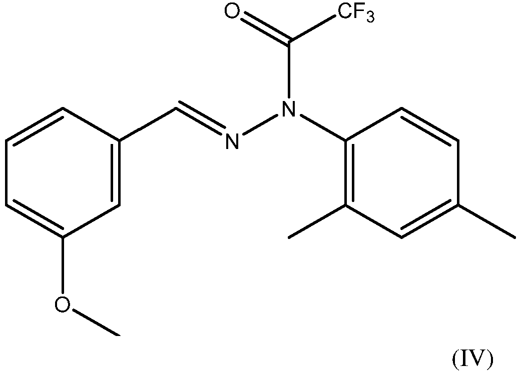

The structure of Formula IV is sometimes referred to herein as "J147".

The following terms have the respective definitions set out below.

"Alkyl" refers to straight or branched chain alkyl radicals having in the range of about 1 up to about 12 carbon atoms (e.g., methyl, ethyl, propyl, butyl, and the like). "Substituted alkyl" refers to alkyl further bearing one or more substituents (*e.g*., 1, 2, 3, 4, or even 5) as set forth herein. "Optionally substituted alkyl" refers to alkyl or substituted alkyl.

"Cycloalkyl" refers to cyclic ring-containing groups containing in the range of about 3 up to about 12 carbon atoms. "Substituted cycloalkyl" refers to cycloalkyl further bearing one or more substituents (*e.g*., 1, 2, 3, 4, or even 5) selected from alkyl, substituted alkyl, as well as any of the substituents set forth herein. "Optionally substituted cycloalkyl" refers to cycloalkyl or substituted cycloalkyl.

"Heterocycle," "heterocyclic" and like terms refer to cyclic (i.e., ring-containing) groups containing one or more heteroatoms (*e.g*., N, O, S, or the like) as part of the ring, and having in the range of 1 up to about 14 carbon atoms. "Substituted heterocyclic" and like terms refer to heterocycle further bearing one or more substituents (*e.g*., 1, 2, 3, 4, or even 5) as set forth herein. Exemplary heterocyclic moieties include saturated rings, unsaturated rings, and aromatic heteroatom-containing ring systems, *e.g*., epoxy, tetrahydrofuran, oxazoline, pyrrole, pyridine, furan, and the like. "Optionally substituted heterocycle" and like terms refer to heterocycle or substituted heterocycle.

Reference to "optionally substituted bicyclic ring" refers to a bicyclic ring structure as known in the art, optionally including substitutions as defined herein.

"Alkenyl" refers to straight, branched chain, or cyclic hydrocarbyl groups including from 2 to about 20 carbon atoms having at least one, 1-3, 1-2, or one, carbon to carbon double bond. "Substituted alkenyl" refers to alkenyl substituted at 1 or more, *e.g.,* 1, 2, 3, 4, or even 5 positions, with substitution as described herein. "Optionally substituted alkenyl" refers to alkenyl or substituted alkenyl. In some embodiments, an alkenyl is ethylenyl or propylenyl. In certain embodiments, a substituted alkenyl is a substituted ethylenyl or substituted propylenyl. In some embodiments, ethylenyl or propylenyl is substituted with one or more CN moieties. For example, in some embodiments, a substituted ethylenyl comprises (CN)₂C=CH-.

"Aryl" refers to aromatic groups having in the range of 6 up to about 14 carbon atoms. "Substituted aryl" refers to aryl radicals further bearing one or more substituents (*e.g*., 1, 2, 3, 4, or even 5) selected from alkyl, substituted alkyl, cycloalkyl, substituted cycloalkyl, hydroxyl, alkoxy, aryloxy, mercapto, alkylthio, arylthio, carbonyl, aryl, substituted aryl, heterocyclic, substituted heterocyclic, halogen, trifluoromethyl, pentafluoroethyl, cyano, cyanoalkyl, nitro, amino, amido, amidino, carboxyl, carbamate, SO₂X, wherein X is H, R, NH₂, NHR or NR₂, SO₃Y, wherein Y is H, NH₂, NHR or NR₂, or C(O)Z, wherein Z is OH, OR, NH₂, NHR or NR₂, and the like. "Optionally substituted aryl" refers to aryl or substituted aryl.

"Aralkyl" refers to an alkyl group substituted by an aryl group. "Substituted aralkyl" refers to aralkyl further bearing one or more substituents (*e.g*., 1, 2, 3, 4, or even 5) selected from alkyl, substituted alkyl, cycloalkyl, substituted cycloalkyl, as well as any of the substituents set forth herein. Thus, aralkyl groups include benzyl, diphenylmethyl, and 1-phenylethyl (-CH(C₆H₅)(CH₃)) among others. "Optionally substituted aralkyl" refers to aralkyl or substituted aralkyl.

"Heteroaryl" refers to aromatic groups containing one or more heteroatoms (e.g., N, O, S, or the like) as part of the aromatic ring, typically having in the range of 2 up to about 14 carbon atoms, and "substituted heteroaryl" refers to heteroaryl radicals further bearing one or more substituents (*e.g*., 1, 2, 3, 4, or even 5) selected from alkyl, substituted alkyl, cycloalkyl, substituted cycloalkyl, as well as any of the substituents set forth above.

"Heteroaralkyl" and "heteroarylalkyl" refer to an alkyl group substituted by one or more heteroaryl groups. "Substituted heteroaralkyl" refers to heteroaralkyl further bearing one or more substituents (*e.g*., 1, 2, 3, 4, or even 5) selected from alkyl, substituted alkyl, cycloalkyl, substituted cycloalkyl, as well as any of the substituents set forth herein. "Optionally substituted heteroaralkyl" refers to heteroaralkyl or substituted heteroaralkyl.

"Halogen" and "halo" refer to fluorine, chlorine, bromine or iodine.

"Hydroxyl" and "hydroxy" refer to the functionality -OH.

"Alkoxy" denotes the group -OR, where R is alkyl. "Substituted alkoxy" denotes the group -OR, where R is substituted alkyl. "Optionally substituted alkoxy" refers to alkoxy or substituted alkoxy.

"Aryloxy" denotes the group -OR, where R is aryl. "Substituted aryloxy" denotes the group -OR, where R is substituted aryl. "Optionally substituted aryloxy" refers to aryloxy or substituted aryloxy.

"Mercapto" and "thiol" refer to the functionality -SH.

"Alkylthio" and "thioalkoxy" refer to the group -SR, -S(O)ₙ=₁₋₂-R, where R is alkyl. "Substituted alkylthio" and "substituted thioalkoxy" refers to the group -SR, -S(O)ₙ=₁₋₂-R, where R is substituted alkyl. "Optionally substituted alkylthio" and "optionally substituted thioalkoxy" refers to alkylthio or substituted alkylthio.

"Arylthio" denotes the group -SR, where R is aryl. "Substituted arylthio" denotes the group -SR, where R is substituted aryl. "Optionally substituted arylthio" refers to arylthio or substituted arylthio.

"Amino" refers to unsubstituted, monosubstituted and disubstituted amino groups, including the substituent -NH₂, "monoalkylamino," which refers to a substituent having structure -NHR, wherein R is alkyl or substituted alkyl, and "dialkylamino," which refers to a substituent of the structure -NR₂, wherein each R is independently alkyl or substituted alkyl.

"Amidino" denotes the group -C(=NR^{q})NR^{r}R^{s}, wherein R^{q}, R^{r.}, and R^{s} are independently hydrogen or optionally substituted alkyl.

Reference to "amide group" embraces substituents of the structure -C(O)-NR₂, wherein each R is independently H, alkyl, substituted alkyl, aryl or substituted aryl as set forth above. When each R is H, the substituent is also referred to as "carbamoyl" (i.e., a substituent having the structure -C(O)-NH₂). When only one of the R groups is H, the substituent is also referred to as "monoalkylcarbamoyl" (i.e., a substituent having the structure -C(O)-NHR, wherein R is alkyl or substituted alkyl as set forth above) or "arylcarbamoyl" (i.e., a substituent having the structure -C(O)-NH(aryl), wherein aryl is as defined above, including substituted aryl). When neither of the R groups are H, the substituent is also referred to as "di-alkylcarbamoyl" (i.e., a substituent having the structure -C(O)-NR₂, wherein each R is independently alkyl or substituted alkyl as set forth above).

Reference to "carbamate" embraces substituents of the structure -O-C(O)-NR₂, wherein each R is independently H, alkyl, substituted alkyl, aryl or substituted aryl.

Reference to "ester group" embraces substituents of the structure -O-C(O)-OR, wherein each R is independently alkyl, substituted alkyl, aryl or substituted aryl.

"Acyl" refers to groups having the structure -C(O)R, where R is hydrogen, alkyl, aryl, and the like as defined herein. "Substituted acyl" refers to acyl wherein the substituent R is substituted as defined herein. "Optionally substituted acyl" refers to acyl and substituted acyl.

"Cyanoalkyl" refers to the group -R≡N, wherein R is an optionally substituted alkylenyl.

As used here, "substitution" denotes an atom or group of atoms that has been replaced with another atom or group of atoms (i.e., substituent), and includes all levels of substitution, *e.g*. mono-, di-, tri-, tetra-, penta-, or even hex-substitution, where such substitution is chemically permissible. Substitutions can occur at any chemically accessible position and on any atom, such as substitution(s) on carbon and any heteroatom, such as oxygen, nitrogen, or sulfur. For example, substituted moieties include those where one or more bonds to a hydrogen or carbon atom(s) contained therein are replaced by a bond to non-hydrogen and/or non-carbon atom(s). Substitutions can include, but are not limited to, a halogen atom such as F, Cl, Br, and I; an oxygen atom in groups such as hydroxyl groups, alkoxy groups, aryloxy groups, and ester groups; a sulfur atom in groups such as thiol groups, alkyl and aryl sulfide groups, sulfone groups, sulfonyl groups, and sulfoxide groups; a nitrogen atom in groups such as amines, amides, alkylamines, dialkylamines, arylamines, alkylarylamines, diarylamines, N-oxides, imides, and enamines; a silicon atom in groups such as trialkylsilyl groups, dialkylarylsilyl groups, alkyldiarylsilyl groups, and triarylsilyl groups; and heteroatoms in other groups as well known in the art.

Non-limiting examples of substituents include, without limitation, halogen, -OH, - NH₂, -NO₂, -CN, -C(O)OH, -C(S)OH, -C(O)NH₂, -C(S)NH₂, -S(O)₂NH₂, -NHC(O)NH₂, - NHC(S)NH₂, -NHS(O)₂NH₂, -C(NH)NH₂, -OR, -SR, -OC(O)R, -OC(S)R, -C(O)R, - C(S)R, -C(O)OR, -C(S)OR, -S(O)R, -S(O)₂R, -C(O)NHR, -C(S)NHR, -C (O)NRR, - C(S)NRR, -S(O)₂NHR, -S(O)₂NRR, -C(NR)NHR, -C(NH)NRR, -NHC(O)R, -NHC(S)R, - NRC(O)R, -NRC(S)R, -NHS(O)₂R, -NRS(O)₂R, -NHC(O)NHR, -NHC(S)NHR, - NRC(O)NH₂, -NRC(S)NH₂, -NRC(O)NHR, -NRC(S)NHR, -NHC(O)NRR, -NHC(S)NRR, -NRC(O)NRR, -NRC(S)NRR, -NHS(O)₂NHR, -NRS(O)₂NH₂, -NRS(O)₂NHR, - NHS(O)₂NRR, -NRS(O)₂NRR, -NHR, -NRR, where R at each occurrence is independently H, optionally substituted alkyl, optionally substituted aryl, or optionally substituted heteroaryl. Also contemplated is substitution with an optionally substituted hydrocarbyl moiety containing one or more of the following chemical functionalities: -O-, -S-, -NR-, - O-C(O)-, -O-C(O)-O-, -O-C(O)-NR-, -NR-C(O)-, -NR-C(O)-O-, -NR-C(O)-NR-, -S-C(O)-, -S-C(O)-O-, -S-C(O)-NR-, -S(O)-, -S(O)₂-, -O-S(O)₂-, -O-S(O)₂-O, -O-S(O)₂-NR-, -O-S(O)-, -O-S(O)-O-, -O-S(O)-NR-, -O-NR-C(O)-, -O-NR-C(O)-O-, -O-NR-C(O)-NR-, - NR-O-C(O)-, -NR-O-C(O)-O-, -NR-O-C(O)-NR-, -O-NR-C(S)-, -O-NR-C(S)-O-, -O-NR-C(S)-NR-, -NR-O-C(S)-, -NR-O-C(S)-O-, -NR-O-C(S)-NR-, -O-C(S)-, -O-C(S)-O-, -O-C(S)-NR-, -NR-C(S)-, -NR-C(S)-O-, -NR-C(S)-NR-, -S-S(O)₂-, -S-S(O)₂-O-, -S-S(O)₂-NR-, -NR-O-S(O)-, -NR-O-S(O)-O-, -NR-O-S(O)-NR-, -NR-O-S(O)₂-, -NR-O-S(O)₂-NR-, -O-NR-S(O)-, -O-NR-S(O)-O-, -O-NR-S(O)-NR-, -O-NR-S(O)₂-O-, -O-NR-S(O)₂-NR-, - O-NR-S(O)₂-, -O-P(O)R₂-, -S-P(O)R₂-, or -NRP(O)R₂-, where R at each occurrence is independently H, optionally substituted alkyl, optionally substituted aryl, or optionally substituted heteroaryl.

In some embodiments, a compound of the invention includes isomers including stereoisomers (*e.g*., enantiomer and diasteromers), tautomers, conformational isomers, and geometric isomers of a compound disclosed herein.

Exemplary conformational isomers include for example without limitation, isomers produced by rotation about a bond wherein the rotation is hindered to the extent that separable isomers result, as well known in the art.

Exemplary geometrical isomers include double bonds in *e.g.,* the "E" or "Z" configuration, as well known in the art.

Compounds of the invention can be readily prepared using a suitable synthetic method. For example, curcumin can be condensed with phenyl hydrazine by warming to reflux overnight in toluene. Optionally, a catalytic amount of acid (HC1) can be employed. In some embodiments, pure curcumin (vs. technical grade) and freshly distilled phenyl hydrazine can be employed.

As another example, 3-methoxy benzaldehyde can be condensed with 2,4-dimethylphenyl hydrazine in methanol employing standard hydrazone preparation conditions (*e.g.,* heating in the microwave to speed the reaction time). Next, the free NH is acylated with TFAA (trifluoroacetic anhydride) plus catalytic (0.1%) amounts of DMAP (dimethylamino pyridine), THE (tetrahydrofuran) or DCM (dichloromethane).

In some embodiments, CF₃ substituted triazoles can be prepared by 1,3-dipolar cycloaddition between suitable aryltrifluoromethylacetylenes and aryl azides. Regioselectivity can be obtained by utilizing a suitable click chemistry (*e.g.,* see Huisgen R. (1984) 1,3-Dipolar Cycloaddition Chemistry, pp. 1-176, Lodon:Wiley; Padwa (1991) Comprehensive Organic Synthesis, Vol. 4:pp. 1069-1109, Oxford: Pergamon; and Fan & Katritzky (1996) Comprehensive Heterocyclic Chemistry II, Vol. 4:pp. 101-126, Oxford: Pergamon). Additional methods of generating compounds disclosed herein can be found in Lima et al., (2015) Chem. Commun. 51:10784-10796 and Kim et al., (2015) Org. Biomol. Chem. 13:9564-9569.

In some embodiments, a compound of the invention is provided in the form of pharmaceutically acceptable salt. A compound of the invention can be complexed with any suitable inorganic or organic salt. In some embodiments, a salt of a compound of the invention is prepared by reacting a compound of the invention with a suitable organic or inorganic acid or base. Non-limiting examples of organic salts contemplated for use herein with a compound of the invention include methanesulfonate, acetate, oxalate, adipate, alginate, aspartate, valerate, oleate, laurate, borate, benzoate, lactate, phosphate, toluenesulfonate (tosylate), citrate, malate, maleate, fumarate, succinate, tartrate, napsylate, methanesulfonate, 2-naphthalenesulfonate, nicotinate, benzenesulfonate, butyrate, camphorate, camphorsulfonate, cyclopentanepropionate, digluconate, dodecylsulfate, glucoheptanoate, glycerophosphate, heptanoate, hexanoate, undecanoate, 2-hydroxyethanesulfonate, ethane sulfonate, and the like. In some embodiments, inorganic salts can be formed from inorganic acids such as sulfate, bisulfate, hemisulfate, hydrochloride, chlorate, perchlorate, hydrobromide, hydroiodide, and the like. Non-limiting examples of a base salt include ammonium salts; alkali metal salts such as sodium salts, potassium salts, and the like; alkaline earth metal salts such as calcium salts, magnesium salts, and the like; salts with organic bases such as dicyclohexylamine salts, N-methyl-D-glucamine, phenylethylamine, and the like; and salts with amino acids such as arginine, lysine, and the like. Salt forms of a compound of the invention can be prepared employing a suitable method.

### POCD

Presented herein is the compound of the invention for use in methods of preventing, reducing, delaying and/or treating a post-operative cognitive dysfunction (POCD), or one or more symptoms thereof, which methods, in certain embodiments, comprise administering a therapeutically effective amount of a compound of the invention, or a pharmaceutical composition comprising a compound of the invention, to a subject *(e.g.,* a subject in need thereof). In certain embodiments, POCD is a new cognitive impairment or decline in cognitive function arising after a medical procedure, which can be diagnosed by comparing the results of pre- and post-operative cognitive tests. According to the invention, POCD is an impairment or decline of cognitive function induced by a medical procedure *(e.g.,* a major surgery). In some embodiments, POCD is a chronic impairment or decline of cognitive function (i.e., chronic POCD) that can be prevented or treated *(e.g.,* reduced, delayed, and/or eliminated) by a method disclosed herein. In some embodiments, chronic POCD refers to a chronic impairment of cognitive function or decline in cognitive function induced by a medical procedure that may last for more than 6 months, more than 12 months or more than 18 months in the absence of treatment. Accordingly, in some embodiments, a method comprises preventing or treating chronic post-operative cognitive dysfunction (POCD), or one or more symptoms thereof, in a subject comprising administering to the subject a therapeutically effective amount of a compound disclosed herein. In some embodiments, POCD is an acute impairment of cognitive function or acute decline in cognitive function (i.e., acute POCD) that can be prevented and/or treated *(e.g.,* reduced, delayed, and/or eliminated) by a method disclosed herein. In some embodiments, acute POCD is an impairment of cognitive function or decline in cognitive function induced by a medical procedure, which may last up to 6 months, up to 12 months, up to 20 months, or up to 36 months following a medical procedure. Accordingly, in some embodiments, a method comprises preventing or treating acute post-operative cognitive dysfunction (POCD), or one or more symptoms thereof, in a subject comprising administering to the subject a therapeutically effective amount of a compound disclosed herein. Additional non-limiting examples of a post-operative cognitive dysfunction include post-operative loss of memory, post-operative loss of concentration, post-operative delirium, a post-operative acute confusional state, post-operative reduced awareness, and a post-operative decline in cognitive function, all of which are induced by, or are the result of a medical procedure. In some embodiments, POCD refers to a pre-existing cognitive condition that is exacerbated or made worse by a medical procedure. In some embodiments POCD refers to an acute or chronic exacerbation of, and/or worsening of a pre-existing (e.g., pre-diagnosed) neurodegenerative disease that is exacerbated and/or made worse by a medical procedure.

In certain embodiments, a method comprises preventing, reducing the severity of, reducing the frequency of, delaying the onset of, or eliminating one or more symptoms of POCD, where the method comprises administering to a subject a therapeutically effective amount of a compound of the invention. Non-limiting examples of symptoms of POCD include a motor or cognitive deficiency; fatigue (e.g., excessive fatigue); passivity; lethargy; inertia; tremors; ataxia; speaking difficulty *(e.g.,* slurred, thick or irregular speech); muscle cramps *(e.g.,* excessive muscle cramping, not necessarily induced by excessive use or excessive exercise), twitching, atrophy or weakness; shortness of breath; breathing difficulty; short term memory loss; long term memory loss; difficulty concentrating; difficulty completing familiar or routine tasks; space and time confusion; vision, color or sign recognition loss; depth perception loss, writing difficulty; loss of reading comprehension; loss of judgment; vocabulary loss; moodiness; unusual or frequent irritability; unusual or frequent aggression; paranoia; delusions; withdrawal from social engagement; unusual or frequent stiffness or rigidity; loss of fine or gross motor control; slowing of movement; impaired balance; body instability; posture or gait abnormality (*e.g*., shuffling walk, unsteady or irregular gait); reduced coordination; motor dysfunction; jerky or involuntary body movement; slowed saccadic eye movement; seizures; difficulty chewing, eating, or swallowing; deterioration in cognition/mental capabilities; dementia; irregular sleep, insomnia, sleep disruption; diagnosed behavioral or psychiatric abnormalities; impaired regulation of social conduct; social withdrawal; over-activity; pacing; wandering; loss of balance; lunging forward when mobilizing; fast walking; imbalance; falls; changes in personality; loss of inhibition or ability to organize information; opthalmoparesis or impaired eye movement; impaired eyelid function; involuntary facial muscle contracture; neck dystonia or backward tilt of the head with stiffening of neck muscles; urinary/bowel incontinence; parkinsonism; the like and combinations thereof. In some embodiments, a method comprises treating and/or preventing post-operative memory impairment or post-operative memory loss by administering a compound disclosed herein. In some embodiments, a method comprises treating and/or preventing a post-operative decline in, or loss of attention by administering a compound disclosed herein. In some embodiments, a method comprises treating and/or preventing a post-operative decline in, or loss of concentration by administering a compound disclosed herein.

In some embodiments, a method comprises preventing, reducing, delaying or treating a post-operative akinetic crisis, or one or more symptoms thereof, in a subject comprising administering to the subject a therapeutically effective amount of a compound of the invention. Post-operative akinetic crisis (also known as acute akinesia), refers to a situation when motor symptoms of Parkinson's Disease (PD) have acutely worsened as a result of a medical procedure *(e.g.,* a major surgery), up to a point when the patient is nearly completely akinetic. Non-limiting examples of symptoms of post-operative akinetic crisis also include worsening of, or a presentation of dysphagia, hyperthermia, dysautonomia, tremors, bradykinesia, muscle rigidity, loss of movement, difficulty with bodily movements, slowed movement, impaired posture and balance, speech changes, writing changes, micrographia, stiff muscles, difficulty standing, difficulty walking, involuntary movements, problems with coordination, rhythmic muscle contractions, increased levels of serum muscle enzymes, the like and combinations thereof.

In certain embodiments, a method comprises preventing, reducing, delaying or treating post-operative delirium, or one or more symptoms thereof, in a subject comprising administering to the subject a therapeutically effective amount of a compound of the invention. Non-limiting examples of symptoms of post-operative delirium include reduced awareness of a subject's environment, a decrease in the ability to focus attention, disorientation of time, place and person, language disturbance (*e.g*., inability to name objects, inability to write, and rambling speech), perceptual disturbances (e.g., hallucinations, illusions or misinterpretations), the like and combinations thereof.

In certain embodiments, a method comprises preventing, reducing, delaying or treating a post-operative acute confusional state in a subject comprising administering to the subject a therapeutically effective amount of a compound of the invention. In certain embodiments, a method comprises preventing, reducing, delaying or treating a post-operative reduced awareness in a subject comprising administering to the subject a therapeutically effective amount of a compound of the invention. In certain embodiments, a method comprises preventing, reducing, delaying or treating a post-operative decline in cognitive function in a subject comprising administering to the subject a therapeutically effective amount of a compound of the invention.

In certain embodiments, a method comprises preventing, reducing, delaying or treating a post-operative central anticholinergic syndrome in a subject comprising administering to the subject a therapeutically effective amount of a compound of the invention.

In certain embodiments, a method comprises preventing or treating an post-operative acute exacerbation of, and/or worsening of a pre-existing (e.g., pre-diagnosed) neurodegenerative disease.

The term "post-operative" as used herein refers to a condition occurring or observed after a medical procedure or surgery is conducted. A post-operative condition is a condition that is perceived to be, or determined to be *(e.g.,* by a medical professional), induced by, caused by or worsened by a medical procedure or surgery conducted.

POCD can be detected and/or diagnosed by comparing the results of a cognitive test *(e.g.,* a psychometric test) conducted before and after a medical procedure. Therefore, POCD is often a new cognitive impairment or cognitive dysfunction detected after a medical procedure. Accordingly, in certain embodiments, POCD is a cognitive impairment or cognitive dysfunction that is not present prior to a medical procedure, but is present after a medical procedure is conducted. In some embodiments, subjects exhibiting a drop in cognitive performance after a medical procedure, compared to before the medical procedure, in one or more cognitive tests can be defined as having POCD. In some embodiments, POCD is a cognitive impairment, cognitive dysfunction, or one or more symptoms thereof, that is enhanced, progressed or made worse after a medical procedure, compared to an assessment of the same condition prior to the medical procedure. Post-operative cognitive testing can be performed at least 1 day, or at least at least 1 week after a medical procedure to allow a patient to recover from the acute effects of anesthesia. In some embodiments, a first post-operative cognitive test is performed at 1 day to 30 days, 1 day to 15 days, or 1 day to 7 days after a medical procedure. In some embodiments, a first preoperative cognitive test is performed at 1 to 6 months, 1 day to 30 days, 1 day to 15 days, or 1 day to 7 days prior to a medical procedure.

Multiple cognitive domains can be tested, non-limiting examples of which include learning, memory, attention and concentration. Non-limiting examples of cognitive tests that can be performed to diagnose the presence, absence or amount of POCD include the Mini-Mental State Examination (MMSE)(*e.g*., see Saczynski et al., (2012) N. Engl. J. Med. 367:30-39); the Reliable Change Index *(e.g.,* see Lewis et al., (2006) Acta Anaesthesiol Scand. 50:50-57; and Berger et al., (2015) Anesthesiol Clin. 33(3):517-50); the Rey Auditory Verbal Learning Tests; Trail Making Tests, Parts A & B; the Grooved Peg Board Test; the Digit Span Tests; the Stroop Tests, the Four-Field Tests, Erzigkeit's Short Cognitive Performance Test; a patients self-assessment; as well as a variety of tests disclosed in various clinical trials *(e.g.,* see ClinicalTrials.gov Identifier: NCT0361019, NCT03540433, NCT02265263, NCT02650687, NCT02848599, NCT03084393, NCT03029676 and NCT03635229).

POCD is not clearly tied to any pathological process and the etiology of POCD has not been determined. Certain studies speculate that inflammation may play a role in POCD. However such studies have provided inconsistent results. Further, several immunosuppressive/anti-inflammatory drugs have failed to prevent or treat POCD. For example, Magnesium (considered an immunosuppressive agent), administered intravenously during cardiac surgery failed to reduce POCD in a clinical trial *(e.g.,* see ClinicalTrials.gov Identifier: NCT00041392). As another example, Pexelizumab, a humanized monoclonal antibody used as an immunosuppressive drug, had no effect on POCD after coronary artery bypass graft surgery *(e.g.,* Mathew et al. (2004) Stroke 35:2335-239). As yet another example, Minocycline, an antibiotic shown to have anti-inflammatory properties and neuroprotective effects, exacerbated POCD (Li W, et al., (2018) J Int Med Res. 46(4):1404-1413). Accordingly, one cannot predict with any reasonable certainty that POCD is a disorder caused by inflammation or that a particular agent that has anti-inflammatory or immunosuppressive properties can be used to prevent or treat POCD.

Also, several drugs that are used to treat neurodegenerative diseases have failed to prevent or treat POCD. For example, donepezil (Aricept), a drug used to treat dementia, memory loss and Alzheimer's disease, had no effect on the overall cognitive index in patients with cognitive decline one year after cardiac surgery (Doraiswamy et al., (2007) Psychopharmacol Bull. 40:54-62). Dexmedetomidine, a sedative suggested for use in treating delirium (MacLaren, et al. (2015) Journal of Intensive Care Medicine. 30 (3): 167-175) also failed to show efficacy for POCD (Skvarc et al., (2018) Neurosci. Biobehav. Rev. 84, 116-133). A clinical investigation testing the use of Rivastigmine (Exelon), an AD/Parkinson's drug, for the treatment of POCD was terminated because it was found to be too dangerous in seriously ill patients after surgery, and at termination, no efficacy was observed (NCT00835159). Accordingly, one cannot predict with any reasonable certainty that a drug used to successfully treat a neurogenerative disease can be also be used to prevent or treat POCD.

### Subjects

The term "subject" refers to a mammal. Any suitable mammal can be treated by a method or composition described herein. Non-limiting examples of mammals include a human, non-human primate *(e.g.,* ape, gibbons, chimpanzees, orangutans, monkeys, macaques, and the like), domestic animals *(e.g.,* dogs and cats), farm animals *(e.g.,* horses, cows, goats, sheep, pigs) and experimental animals (*e.g*., mouse, rat, rabbit, guinea pig). In some embodiments a subject is a non-human primate or a human. In some embodiments a subject is a human. A subject can be any age or at any stage of development (*e.g*., an adult, teen, child, infant, or a mammal in utero). A subject can be male or female.

In some embodiments, a subject is a subject displaying stable cognitive function (*e.g*., prior to an impending medical procedure). In some embodiments a subject has not previously been diagnosed with a cognitive disorder or neurodegenerative disease (*e.g*., prior to an impending medical procedure). In some embodiments a subject has not previously been diagnosed with cancer, diabetes, arthritis, insulinoma, stroke, or ischemia (*e.g*., heart ischemia). In some embodiments a subject was not previously administered a compound selected from any one of Formula I, Formula II, Formula III and Formula IV. In certain embodiments a subject is about to have, is scheduled to have, is having and/or has recently had (*e.g*., within hours to 1-7 days) a medical procedure.

In certain embodiments, a subject is at risk of developing a post-operative cognitive dysfunction. In some embodiments, a subject at risk of developing a post-operative cognitive dysfunction is 45 years old or older, 50 years old or older, 55 years old or older, 60 years old or older, 65 years old or older, 70 years old or older, or 75 years old or older.

In certain embodiments, a subject at risk of developing a post-operative cognitive dysfunction is a subject previously diagnosed with a cognitive disorder or neurodegenerative disease (*e.g*., prior to an impending medical procedure). Non-limiting examples of a neurodegenerative disease includes Alzheimer's disease, Parkinson's disease, Huntington's disease, amyotrophic lateral sclerosis, glaucoma, retinal degeneration, macular degeneration, age-related hearing loss, mild cognitive impairment, dementia, progressive supranuclear palsy, spinocerebellar ataxia, retinal neuropathy, peripheral neuropathy, diabetic neuropathy, background neuropathy, familial amyloid polyneuropathy, systemic senile amyloidosis, prion disease, scrapie, bovine spongiform encephalopathy, Creutzfeldt-Jakob disease, Gerstmann-Straussler-Scheinker syndrome and amyloidosis.

In certain embodiments, a subject at risk of developing a post-operative cognitive dysfunction is a subject with an educational level equivalent to 12^{th} grade (i.e., high school GED or diploma) or less. In certain embodiments, a subject at risk of developing a post-operative cognitive dysfunction is a subject with a history of excessive alcohol intake or substance abuse, and/or is, or was diagnosed as an alcoholic. In certain embodiments, a subject at risk of developing a post-operative cognitive dysfunction is a subject suffering from depression, a subject previously diagnosed with depression, or a subject prone to depression. In certain embodiments, a subject at risk of developing a post-operative cognitive dysfunction is a subject diagnosed with high blood pressure or hypertension. In some embodiments, high blood pressure means a systolic pressure of 130 (mm Hg) or higher and/or a diastolic pressure of 80 (mm Hg) or higher.

### Medical Procedures

In some embodiments, a medical procedure comprises minor and/or major surgery. In certain embodiments, a surgery is an invasive medical process involving a surgical incision. A medical procedure may involve the use of a local or general anesthetic. Non-limiting examples of major surgery include a surgery that involves opening the peritoneal cavity, a surgery that involves breaching the skull, a surgery involving a risk of severe hemorrhage, a surgery where the life of a patient is at risk, a surgery involving the full or partial removal of, replacement of, or repair of a major organ *(e.g.,* heart, lung, kidney, stomach, intestine, bone, brain, stomach, pancreas, gall bladder, appendix, colon, and liver), the like and combinations thereof. In some embodiments, a major surgery is surgical procedure having a duration of at least 0.3 hours, at least 0.4 hours, at least 0.5 hours, at least 1 hour, at least 1.5 hours, at least 2 hours, or at least 2.5 hours (*e.g*., as measured from an initial incision to a closing suture). In some embodiments, a major surgery is surgical procedure having a duration of 0.5 hours to 20 hours, or 1 hour to 15 hours. In some embodiments a medical procedure is a surgery requiring general anesthesia for at least 0.5 hours, at least1 hour, at least 1.5 hours or at least 2 hours. Additional non-limiting examples of a major surgery include cardiac surgery, angioplasty, organ transplant surgery, complete or partial removal of an organ or tissue, brain surgery, bone replacement or repair surgery (*e.g*., knee replacement surgery, or hip replacement surgery, and the like), arthrodesis, bypass surgery, hernia repair, abdominal surgery, bariatric surgery, facial reconstruction, breast reconstruction surgery, replantation surgery, exploratory surgery, amputation, and pelvic floor surgery. In some embodiments, a major surgery is a surgery that requires a subject to be on a ventilator or heart-lung bypass machine during surgery.

In some embodiments a medical procedure comprises minor surgery. In some embodiments, a minor surgery is a surgery requiring only a local anesthetic. In some embodiments a minor surgery is a surgery that does not require general anesthesia. In some embodiments, a minor surgery is surgical procedure having a duration of less than 0.3 hours, less than 0.4 hours, less than 0.5 hours, less than 1 hour, less than 1.5 hours, less than 2 hours, or less than 2.5 hours (*e.g.,* as measured from an initial incision to a closing suture). In some embodiments, a minor surgery is a surgical procedure having a duration of 0.01 hours to 3 hours, 0.01 hours to 2 hours, 0.01 hours to 1.5 hours, 0.1 hours to 3 hours, 0.1 hours to 2 hours, or 0.1 hours to 1.5 hours.

### Pharmaceutical compositions

In some embodiments, a composition or pharmaceutical composition comprises a compound of the invention. In some embodiments, a composition or pharmaceutical composition comprises a therapeutically effective amount of a compound of the invention. In some embodiments, a composition or pharmaceutical composition comprises a compound disclosed herein in an amount in a range of 1 µg to 100 mg, or 10 µg to 100 µg. In some embodiments provided herein is a pharmaceutical composition comprising a compound of the invention for use in preventing or treating POCD, or one or more symptoms thereof. In some embodiments, a pharmaceutical composition comprises a compound of the invention and a pharmaceutically acceptable excipient, diluent, additive or carrier.

A pharmaceutical composition can be formulated for a suitable route of administration. In some embodiments a pharmaceutical composition is formulated for oral, subcutaneous (s.c.), intradermal, intramuscular, intraperitoneal and/or intravenous (i.v.) administration. In certain embodiments, a pharmaceutical composition contains formulation materials for modifying, maintaining, or preserving, for example, the pH, osmolarity, viscosity, clarity, color, isotonicity, odor, sterility, stability, rate of dissolution or release, adsorption or penetration of the composition. In certain embodiments, suitable formulation materials include, but are not limited to, amino acids (such as glycine, glutamine, asparagine, arginine or lysine); antimicrobials; antioxidants (such as ascorbic acid, sodium sulfite or sodium hydrogen-sulfite); buffers (such as borate, bicarbonate, Tris-HCl, citrates, phosphates (*e.g*., phosphate buffered saline) or suitable organic acids); bulking agents (such as mannitol or glycine); chelating agents (such as ethylenediamine tetraacetic acid (EDTA)); complexing agents (such as caffeine, polyvinylpyrrolidone, beta-cyclodextrin or hydroxypropyl-beta- cyclodextrin); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counter ions (such as sodium); solvents (such as glycerin, propylene glycol or polyethylene glycol); diluents; excipients and/or pharmaceutical adjuvants. In particular, a pharmaceutical composition can comprise any suitable carrier, formulation, or ingredient, the like or combinations thereof as listed in "Remington: The Science And Practice Of Pharmacy" Mack Publishing Co., Easton, PA, 19th Edition, (1995)(hereafter, Remington '95), or "Remington: The Science And Practice Of Pharmacy", Pharmaceutical Press, Easton, PA, 22nd Edition, (2013)(hereafter, Remington 2013).

In certain embodiments, a pharmaceutical composition comprises a suitable excipient, non-limiting examples of which include anti-adherents (*e.g*., magnesium stearate), a binder, fillers, monosaccharides, disaccharides, other carbohydrates (*e.g*., glucose, mannose or dextrin), sugar alcohols (*e.g.,* mannitol or sorbitol), coatings (*e.g.,* cellulose, hydroxypropyl methylcellulose (HPMC), microcrystalline cellulose, synthetic polymers, shellac, gelatin, corn protein zein, enterics or other polysaccharides), starch (*e.g.,* potato, maize or wheat starch), silica, colors, disintegrants, flavors, lubricants, preservatives, sorbents, sweeteners, vehicles, suspending agents, surfactants and/or wetting agents (such as pluronics, PEG, sorbitan esters, polysorbates such as polysorbate 20, polysorbate 80, triton, tromethamine, lecithin, cholesterol, tyloxapal), stability enhancing agents (such as sucrose or sorbitol), and tonicity enhancing agents (such as alkali metal halides, sodium or potassium chloride, mannitol, sorbitol), and/or any excipient disclosed in Remington '95 or Remington 2013. The term "binder" as used herein refers to a compound or ingredient that helps keeps a pharmaceutical mixture combined. Suitable binders for making pharmaceutical formulations and are often used in the preparation of pharmaceutical tablets, capsules and granules are known to those skilled in the art.

In some embodiments a pharmaceutical composition comprises a suitable pharmaceutically acceptable additive and/or carrier. Non-limiting examples of suitable additives include a suitable pH adjuster, a soothing agent, a buffer, a sulfur-containing reducing agent, an antioxidant and the like. Non-limiting examples of a sulfur-containing reducing agent include those having a sulfhydryl group *(e.g.,* a thiol) such as N-acetylcysteine, N-acetylhomocysteine, thioctic acid, thiodiglycol, thioethanolamine, thioglycerol, thiosorbitol, thioglycolic acid and a salt thereof, sodium thiosulfate, glutathione, and a C1-C7 thioalkanoic acid. Non-limiting examples of an antioxidant include erythorbic acid, dibutylhydroxytoluene, butylhydroxyanisole, alpha-tocopherol, tocopherol acetate, L-ascorbic acid and a salt thereof, L-ascorbyl palmitate, L-ascorbyl stearate, sodium bisulfite, sodium sulfite, triamyl gallate and propyl gallate, as well as chelating agents such as disodium ethylenediaminetetraacetate (EDTA), sodium pyrophosphate and sodium metaphosphate. Furthermore, diluents, additives and excipients may comprise other commonly used ingredients, for example, inorganic salts such as sodium chloride, potassium chloride, calcium chloride, sodium phosphate, potassium phosphate and sodium bicarbonate, as well as organic salts such as sodium citrate, potassium citrate and sodium acetate.

The pharmaceutical compositions used herein can be stable over an extended period of time, for example on the order of months or years. In some embodiments a pharmaceutical composition comprises one or more suitable preservatives. Non-limiting examples of preservatives include benzalkonium chloride, benzoic acid, salicylic acid, thimerosal, phenethyl alcohol, methylparaben, propylparaben, chlorhexidine, sorbic acid, hydrogen peroxide, the like and/or combinations thereof. A preservative can comprise a quaternary ammonium compound, such as benzalkonium chloride, benzoxonium chloride, benzethonium chloride, cetrimide, sepazonium chloride, cetylpyridinium chloride, or domiphen bromide (BRADOSOL^{®}). A preservative can comprise an alkyl-mercury salt of thiosalicylic acid, such as thimerosal, phenylmercuric nitrate, phenylmercuric acetate or phenylmercuric borate. A preservative can comprise a paraben, such as methylparaben or propylparaben. A preservative can comprise an alcohol, such as chlorobutanol, benzyl alcohol or phenyl ethyl alcohol. A preservative can comprise a biguanide derivative, such as chlorohexidine or polyhexamethylene biguanide. A preservative can comprise sodium perborate, imidazolidinyl urea, and/or sorbic acid. A preservative can comprise stabilized oxychloro complexes, such as known and commercially available under the trade name PURITE^{®}. A preservative can comprise polyglycol-polyamine condensation resins, such as known and commercially available under the trade name POLYQUART^{®} from Henkel KGaA. A preservative can comprise stabilized hydrogen peroxide. A preservative can be benzalkonium chloride. In some embodiments a pharmaceutical composition is free of preservatives.

In some embodiments a composition, pharmaceutical composition or compound of the invention is substantially free of contaminants (*e.g*., blood cells, platelets, polypeptides, minerals, blood-borne compounds or chemicals, virus, bacteria, other pathogens, toxin, and the like). In some embodiments a composition, pharmaceutical composition or compound of the invention is substantially free of serum and serum contaminants (*e.g*., serum proteins, serum lipids, serum carbohydrates, serum antigens and the like). In some embodiments a composition, pharmaceutical composition or compound of the invention is substantially free of a pathogen *(e.g.,* a virus, parasite or bacteria). In some embodiments a composition, pharmaceutical composition or compound of the invention is substantially free of endotoxin. In some embodiments a composition, pharmaceutical composition or compound of the invention is sterile. According to the invention, a composition or pharmaceutical composition disclosed herein comprises a compound of Formula IV.

The pharmaceutical compositions described herein may be configured for administration to a subject in any suitable form and/or amount according to the therapy in which they are employed. For example, a pharmaceutical composition configured for parenteral administration (*e.g*., by injection or infusion), may take the form of a suspension, solution or emulsion in an oily or aqueous vehicle and it may contain formulation agents, excipients, additives and/or diluents such as aqueous or non-aqueous solvents, co-solvents, suspending solutions, preservatives, stabilizing agents and or dispersing agents. In some embodiments a pharmaceutical composition suitable for parenteral administration may contain one or more excipients. In some embodiments a pharmaceutical composition is lyophilized to a dry powder form. In some embodiments a pharmaceutical composition is lyophilized to a dry powder form, which is suitable for reconstitution with a suitable pharmaceutical solvent (*e.g*., water, saline, an isotonic buffer solution (*e.g*., PBS), DMSO, combinations thereof and the like). In certain embodiments, reconstituted forms of a lyophilized pharmaceutical composition are suitable for parenteral administration (*e.g*., intravenous administration) to a mammal.

In certain embodiments, a pharmaceutical composition is configured for oral administration and may be formulated as a tablet, microtablet, minitablets, micropellets, powder, granules, capsules (*e.g*., capsules filled with microtablets, micropellets, powders or granules), emulsions, solutions, the like or combinations thereof. Pharmaceutical compositions configured for oral administration may comprise suitable coatings to delay or sustain release of the active ingredient, non-limiting examples of which include enteric coatings such as fatty acids, waxes, shellac, plastics, methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, cellulose acetate trimellitate, sodium alginate, zein, plant fibers, the like and combinations thereof.

In some embodiments a pharmaceutical compositions described herein may be configured for topical administration and may include one or more of a binding and/or lubricating agent, polymeric glycols, gelatins, cocoa-butter or other suitable waxes or fats. In some embodiments a pharmaceutical composition described herein is incorporated into a topical formulation containing a topical carrier that is generally suited to topical drug administration and comprising any suitable material known to those skilled in the art. In certain embodiments, a topical formulation of a pharmaceutical composition is formulated for administration of a compound of the invention from a topical patch.

In certain embodiments, an optimal pharmaceutical composition is determined by one skilled in the art depending upon, for example, on the intended route of administration, delivery format and desired dosage (see *e.g*., Remington '95 or Remington 2013, supra). A pharmaceutical composition can be manufactured by any suitable manner, including, *e.g*., by means of conventional mixing, dissolving, granulating, dragee-making, levigating, emulsifying, encapsulating, entrapping or tableting processes (*e.g.*, see methods described in Remington '95 or Remington 2013).

### Route of Administration

Any suitable method of administering a composition, pharmaceutical composition or compound of the invention to a subject can be used. Any suitable formulation and/or route of administration can be used for administration of a compound of the invention or composition disclosed herein *(e.g.,* see Fingl et al. 1975, in "The Pharmacological Basis of Therapeutics", which is incorporated herein by reference in its entirety). A suitable formulation and/or route of administration can be chosen by a medical professional (*e.g*., a physician) in view of, for example, a subject's risk, age, and/or condition. Non-limiting examples of routes of administration include topical or local *(e.g.,* transdermally or cutaneously, *(e.g.,* on the skin or epidermis), in or on the eye, intranasally, transmucosally, in the ear, inside the ear *(e.g.,* behind the ear drum)), enteral *(e.g.,* delivered through the gastrointestinal tract, *e.g.,* orally *(e.g.,* as a tablet, capsule, granule, liquid, emulsification, lozenge, or combination thereof), sublingual, by gastric feeding tube, rectally, and the like), by parenteral administration (*e.g.,* parenterally, *e.g.,* intravenously, intra-arterially, intramuscularly, intraperitoneally, intradermally, subcutaneously, intracavity, intracranial, intra-articular, into a joint space, intracardiac (into the heart), intracavernous injection, intralesional (into a skin lesion), intraosseous infusion (into the bone marrow), intrathecal (into the spinal canal), intrauterine, intravaginal, intravesical infusion, intravitreal), the like or combinations thereof.

In some embodiments a compound of the invention or pharmaceutical composition described herein is administered to the lungs, bronchial passages, trachea, esophagus, sinuses, or nasal passages using a suitable method, non-limiting examples of which include intranasal administration, intratracheal instillation, and oral inhalative administration (*e.g*., by use of an inhaler, *e.g*., single/-multiple dose dry powder inhalers, nebulizers, and the like).

In some embodiments a compound of the invention or a pharmaceutical composition disclosed herein is provided to a subject. For example, a composition that is provided to a subject is sometimes provided to a subject for self-administration or for administration to a subject by another *(e.g.,* a non-medical professional). As another example, a composition can be provided as an instruction written by a medical practitioner that authorizes a patient to be provided a composition or treatment described herein *(e.g.,* a prescription). In yet another example, a composition can be provided to a subject where the subject self-administers a composition orally, intravenously or by way of an inhaler, for example.

Alternately, one can administer a compound of the invention or composition in a local rather than systemic manner, for example, via direct application to the skin, mucous membrane or region of interest for treating, including using a depot or sustained release formulation.

In certain embodiments a pharmaceutical composition comprising a compound of the invention is administered alone (*e.g.,* as a single active ingredient (AI or *e.g.,* as a single active pharmaceutical ingredient (API)). In other embodiments, a pharmaceutical composition comprising a compound of the invention is administered in combination with one or more additional AIs/APIs, for example, as two separate compositions or as a single composition where the one or more additional AIs/APIs are mixed or formulated together with a compound of the invention in a pharmaceutical composition.

### Dose and Therapeutically Effective Amount

In some embodiments, an amount of a compound of the invention (*e.g*., in a pharmaceutical composition) is a therapeutically effective amount. In certain embodiments, a pharmaceutical composition comprises a therapeutically effective amount of a compound disclosed herein. In some embodiments, a therapeutically effective amount of a compound of the invention is administered to a subject. In some embodiments, a therapeutically effective amount of a compound of the invention is an amount needed to obtain an effective therapeutic outcome. In certain embodiments, a therapeutically effective amount of a compound of the invention is an amount sufficient to prevent, treat, reduce the severity of, delay the onset of, and/or alleviate one or more symptoms of POCD, post-operative delirium, a post-operative acute confusional state, a post-operative reduced awareness, post-operative central anticholinergic syndrome, post-operative akinetic crisis, or a post-operative decline in cognitive function induced by a medical procedure as contemplated herein. Determination of a therapeutically effective amount is well within the capability of those skilled in the art, especially in light of the detailed disclosure provided herein.

In certain embodiments, a therapeutically effective amount is an amount high enough to provide an effective therapeutic effect *(e.g.,* a beneficial therapeutic effect) and an amount low enough to minimize unwanted adverse reactions. Accordingly, in certain embodiments, a therapeutically effective amount of a compound of the invention may vary from subject to subject, often depending on age, weight, general health condition of a subject, severity of a condition being treated, length of a medical procedure, amount of fluid loss due to trauma or surgery, and/or a particular combination of drugs administered to a subject. Thus, in some embodiments, a therapeutically effective amount is determined empirically. Accordingly, a therapeutically effective amount of a compound of the invention that is administered to a subject can be determined by one of ordinary skill in the art based on amounts found effective in animal or clinical studies, a physician's experience, and suggested dose ranges or dosing guidelines, for example.

In certain embodiments, a therapeutically effective amount of a compound of the invention is administered at a suitable dose *(e.g.,* at a suitable volume, frequency and/or concentration, which often depends on a subject's weight, age and/or condition) intended to obtain an acceptable therapeutic outcome. In certain embodiments, a therapeutically effective amount of a compound of the invention comprises one or more doses selected from at least 0.01 mg/kg *(e.g.,* mg of a compound of the invention per kg body weight of a subject), at least 0.1 mg/kg, at least 0.5 mg/kg, at least 1 mg/kg, at least 10 mg/kg or at least 100 mg/kg. In certain embodiments, a therapeutically effective amount of a compound of the invention is selected from one or more doses of about 0.001 mg/kg *(e.g.,* mg of a compound of the invention per kg body weight of a subject) to about 5000 mg/kg, 0.01 mg/kg to 1000 mg/kg, 0.01 mg/kg to 500 mg/kg, 0.1 mg/kg to 1000 mg/kg, 1 mg/kg to 1000 mg/kg, 10 mg/kg to 1000 mg/kg, 100 mg/kg to 1000 mg/kg, 0.1 mg/kg to 500 mg/kg, 0.1 mg/kg to 250 mg/kg, 0.1 mg/kg to 150 mg/kg, 0.1 mg/kg to 100 mg/kg, 0.1 mg/kg to 75 mg/kg, 0.1 mg/kg to 50 mg/kg, 0.1 mg/kg to 25 mg/kg, 0.1 mg/kg to 10 mg/kg, 0.1 mg/kg to 5 mg/kg, 0.5 mg/kg to 5 mg/kg, intervening amounts and combinations thereof. In some aspects a therapeutically effective amount of a compound of the invention administered to a subject comprises one or more doses of about 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 50 mg/kg, 100 mg/kg, 500 mg/kg, and intervening amounts and combinations thereof. In some embodiments a therapeutically effective amount of a compound of the invention is between about 0.1 mg/kg and about 50 mg/kg.

In some embodiments administering a therapeutically effective amount of a compound of the invention, or a pharmaceutical composition comprising a compound of the invention, comprises administering a suitable dose at a frequency or interval as needed to obtain an effective therapeutic outcome. In some embodiments administering a therapeutically effective amount of a compound of the invention or a pharmaceutical composition disclosed herein comprises administering a suitable dose hourly, every two hours, every 4 hours, every 6 hours, three times a day, twice a day, once a day, six times a week, five times a week, four times a week, three times a week, twice a week, weekly, at combinations thereof, and/or at regular or irregular intervals thereof, and/or simply at a frequency or interval as needed or recommended by a medical professional. In some embodiments, a therapeutically effective amount of a compound of the invention or a pharmaceutical composition comprising a therapeutically effective amount of compound of the invention is administered continuously by, for example by intravenous administration.

In some embodiments a therapeutically effective amount of a compound of the invention is administered to a subject prior to, perioperatively, during and/or after a medical procedure. In some embodiments a therapeutically effective amount of a compound of the invention is administered to a subject up to 3 days prior to, up to 2 days prior to, up to 1 day prior to, up to 20 hours prior to, up to 15 hours prior to, up to 10 hours prior to, up to 5 hours prior to, up to 2 hours prior to or up to 1 hour prior to a medical procedure. In some embodiments a therapeutically effective amount of a compound of the invention is administered to a subject 0 to 72 hours, 0 and 48 hours, 0 to 24 hours, 0 to 12 hours, 0 to 6 hours, 0 to 4 hours, or 0 to 2 hours before a medical procedure. In some embodiments a therapeutically effective amount of a compound of the invention is administered perioperatively. In some embodiments a therapeutically effective amount of a compound of the invention is during a medical procedure. In some embodiments a therapeutically effective amount of a compound of the invention is administered intermittently or continuously for up to 1 hour after, 2 hours after, 4 hours after, 6 hours after, 12 hours after, 24 hours after, 2 days after, 3 days after, a week after, 1 month after, 3 months after, 6 months after, 12 months after, 18 months after, 24 months after or up to 36 months after a medical procedure.

### Kits

In some embodiments, provided herein is a kit comprising a compound of the invention or a pharmaceutical composition comprising a compound of the invention. In some embodiments, a kit comprises one or more doses of a pharmaceutical composition comprising a compound of the invention. In some embodiments, a kit comprises one or more packs and/or one or more dispensing devices, which can contain one or more doses of a compound of the invention, or pharmaceutical composition thereof, as described herein. Non-limiting examples of a pack include a metal, glass, or plastic container, syringe or blister pack that comprises a compound of the invention or a composition described herein. In certain embodiments, a kit comprises a dispensing device such as a syringe or inhaler, that may or may not comprise a compound of the invention or a composition described herein. A pack and/or dispenser device can be accompanied by instructions for administration. The pack or dispenser can also be accompanied with a notice associated with the container in a form prescribed by a governmental agency regulating the manufacture, use, or sale of pharmaceuticals, which notice is reflective of approval by the agency of the form of the drug for human or veterinary administration. Such notice, for example, can be the labeling approved by the U.S. Food and Drug Administration for prescription drugs, or the approved product insert.

In some embodiments a kit or pack comprises an amount of a compound of the invention sufficient to treat a patient for 1 day to 1 year, 1 day to 180 days, 1 day to 120 days, 1 day to 90 days, 1 day to 60 days, 1 day to 30 days, 1-24 hours, 1-12 hours, 1-4 hours, or amount of time there between.

A kit optionally includes a product label and/or one or more packaging inserts, that provide a description of the components or instructions for use *in vitro, in vivo,* or *ex vivo,* of the components therein. Exemplary instructions may include instructions for a treatment protocol or therapeutic regimen. In certain embodiments, a kit comprises packaging material, which refers to a physical structure housing components of the kit. The packaging material can maintain the components sterilely and can be made of material commonly used for such purposes (e.g., paper, corrugated fiber, glass, plastic, foil, ampules, vials, tubes, etc.). Product labels or inserts include "printed matter," *e.g.,* paper or cardboard, or separate or affixed to a component, a kit or packing material *(e.g.,* a box), or attached to an ampule, tube or vial containing a kit component. Labels or inserts can additionally include a computer readable medium, optical disk such as CD- or DVD-ROM/RAM, DVD, MP3, magnetic tape, or an electrical storage media such as RAM and ROM or hybrids of these such as magnetic/optical storage media, FLASH media or memory-type cards. Product labels or inserts can include identifying information of one or more components therein, dose amounts, clinical pharmacology of the active ingredient(s) including mechanism of action, pharmacokinetics (PK) and pharmacodynamics (PD). Product labels or inserts can include information identifying manufacturer information, lot numbers, manufacturer location, date, information on an indicated condition, disorder, disease or symptom for which a kit component may be used. Product labels or inserts can include instructions for the clinician or for a subject for using one or more of the kit components in a method, treatment protocol or therapeutic regimen. Instructions can include dosage amounts, frequency or duration, and instructions for practicing any of the methods, treatment protocols or therapeutic regimes set forth herein. A kit can additionally include labels or instructions for practicing any of the methods described herein. Product labels or inserts can include information on potential adverse side effects and/or warnings.

### EXAMPLES

### Example 1

In this study, the effects of J147 in an animal model of POCD were investigated.

### Materials & Methods

Male Wistar rats (~350 g) were randomly divided into 4 experimental groups; non-surgery; control abdominal surgery; surgery + acute J147 treatment (7 mg/rat by gavage, 4-5 hours before surgery); surgery + chronic J147 (500 mg/kg food, 1week before surgery until the end of the study). Timed blood samples were collected. Days 9-11 post surgery, general behavior (Open field: OF) and cognitive performance (Novel Object/Novel Location Recognition: NOR/NLR; Morris Water Maze: MWM) were tested. Day 14, rats were sacrificed and blood and brain tissue were collected for further analyses. (Neuro)inflammation was examined by circulating cytokine levels and microglia activation in the brain.

### Animals and housing

A total of 60 male Wistar rats (Wu-Wistar, Envigo, The Netherlands) were studied in 5 cohorts. For that, rats were ordered in 5 groups of different weight classes (220-280g) to ensure that each group would be around 350g at the time of surgery. Rats arrived at the facility at least 2 weeks before start of the experiments, and were put on reversed light: dark cycle 12:12 with the lights out at 09:00 am. Rats were housed in groups of 2-3 in standard cages under controlled conditions (temperature of 20+/- 2 degrees and humidity of 50% +/- 10%). The rats were fed with water and Teklad (Envigo, 2018) chow food ad libitum. After surgery, rats were housed individually. Approval of all experiments was given by the applicable animal and welfare committee of the Netherlands.

### Experimental protocol

Within each cohort rats (n=12 per cohort) were equally divided over four experimental groups, including non-surgery, control surgery, surgery + acute J147 and surgery + chronic J147. Half of the non-surgery and control surgery groups received vehicle gavage and served as control for acute treatment.

One week before surgery, chronic treatment J147 treatment started and continued until the end of the experiment. The day of surgery, rats for acute treatment received their J147 (4-5 hours before surgery) by gavage, while the designated control rats received vehicle gavage. Rats were subjected to abdominal surgery, and allowed to recover. After that rats were housed individually. Non-surgery control were placed in individual cages at the same time. Blood samples were collected from the jugular vein catheter at the time of surgery (plasma levels of J147) and at 1, 6 and 24 hours after surgery (plasma inflammatory markers). The first week post-surgery, rats were weighed daily and food intake was measured. Day 7-12 post-surgery rats were subjected to behavioral testing regarding general exploratory behavior and cognitive performance. On Day 14 rats were sacrificed, blood samples were collected from cardiac puncture, CSF was collected, and brain tissue was collected and processed for immunohistochemistry or molecular analyses.

### Acute J147 treatment

For acute oral treatment, crystalline J147 was suspended in "basis for suspension" (Fagron Nederland) at 7 mg/ml, and vortexed until homogenous. On treatment days, a fresh suspension of 1 ml was prepared 30 minutes before gavage and administered using a flexible gavage tube. During preparation gavage containers were covered with aluminum foil with reference to light sensitivity of J147. In accordance with pharmacokinetic studies on oral J147 administration (Abrexa), gavage of J147 suspension was provided 4-5 hours before surgery, when plasma levels reach their maximum values. Control rats received gavage of 1 ml basis for suspension 4-5 hours before the time of surgery.

### Chronic J147 treatment

For chronic oral treatment, J147 was added to the food (Teklad 2018, Envigo) at a concentration of 500 mg/kg. J147 was provided by Abrexa and Envigo prepared the food. The chronically treated animals received J147 food from one week before surgery until sacrifice. The J147 food was administered *ad libitum.* After surgery food intake was measured daily during the last hour of the light phase. J147 food was stored at -20°C (dark). Control rats received Teklad 2018 food (Envigo).

### Surgery

Abdominal surgery was performed as described previously by Hovens *et al.,* 2014. Rats were anaesthetized using sevoflurane (±2,5% in air/O2= 2/1) and received 0.01 mg/kg buprenorphine s.c.. A heating blanket was used to preserve body temperature. An incision was made along the linea alba and the intestines were exteriorised. The upper mesenteric artery was isolated from surrounding tissue and clamped for a period of 30 minutes during which the intestines and surgical wound were covered with gauze soaked in saline, to prevent dehydration. During this time the animals were equipped with an indwelling jugular vein catheter to mimic insertion of a venous line in patients and allow timed blood sampling. For that, an incision was made at the subclavical region, the jugular vein was freed from surrounding tissue and a catheter was introduced and advanced into the vena cava, just above the right atrium. The other end of the catheter was guided subcutaneously to the head, and fixated on the skull with dental cement. The catheter was filled with PVP dissolved in heparin solution and closed with a plastic plug. After subsequent removal of the mesenteric artery clamp, the intestines were gently placed back and the abdominal wall and skin were closed separately by sutures. Clamping and reperfusion were visually verified by absence or presence of pulsation in the mesenteric artery distal to the clamp site. From induction to recovery, the procedure takes approximately 60 minutes. Postoperatively, the animals were allowed to recover and subsequently were housed individually. Rats were weighed daily at the last hour of the dark period. Maximal weight loss within the first 5 days post-surgery was taken as measure of experienced severity of surgery.

### Behavioral tests

Behavioral tests were performed in the dark (active) period of the rats. The lights were turned on at 9:00 p.m. and off at 9:00 a.m. Rats were allowed to eat the first hour of the dark period, as testing does not include the first hour and the last hour of the dark phase. General exploratory behavior and anxiety was tested in the Open field (OF) test; spatial learning, spatial memory and cognitive flexibility in the Morris Water Maze (MWM); spatial working memory in the novel location recognition test (NL); the novel object recognition test (NO) for working memory on object recognition.

### Open field

The Open Field test was performed to assess exploratory and anxiety behavior. The box consisted of a square (100 x 100 x 40 cm) divided into areas: the center area (60 x 60 cm), 4 side areas (20 x 60 cm) and 4 corner areas (20 x 20 cm). The rats were familiarized with the behavioral set-up before the actual behavioral test by letting them freely move in the box for 5 minutes at least 1 hour before the actual test. At the test each rat was in random order placed in the middle of the box and the trial started when the rat crossed one of the lines defining the center area. After five minutes the rat was gently removed from the box and returned to his cage. Before each rat the box was cleaned with 70% ethanol. The movement as well as location of the rat was recorded as time in corner, walls and center, number of visits in the center and the distance walked, and analyzed with Ethovision (Noldus, the Netherlands). Rearing behavior was analyzed by Eline. Exploratory behavior was measured by the distance the rat moved during the test as well as the number of rearings, while anxiety was obtained as reciprocal of the percentage of time spent in center area and number of center visits.

### Novel Object and Novel Location Recognition (NOR/NLR)

The Novel Object and Novel Location Recognition were tested in random order in one procedure to assess spatial short term memory and object memory. For this test, an open square box (50x50x40 cm) with 2 transparent and two grey walls was used. Objects used in the test were selected from a pilot study, investigating the preferences of rats for different objects. Rats were habituated to the box for 3 minutes, the day before the test. The test consisted of four phases, each phase took 3 minutes. Between each phase there was a break of 45 seconds in which the rat remained in the test box. The first phase was performed in an empty box, in the second phase two identical objects were placed in both corners at the nearest end of the box. In this phase, the rat was allowed to explore both objects. After this exploration phase either a novel object test or novel location test was performed. Before the start of each phase both objects were removed from the box and cleaned with 70% ethanol. During the Novel Object (NO) test one of the familiar objects was replaced by a new object and during the Novel Location (NL) test one of the familiar objects was relocated to the top of the box. After each test the box was cleaned with 70% ethanol to remove smell cues before entering of the next animal. The tests were manually analyzed with Eline. Baseline exploratory behavior was calculated by the time the rat spent exploring both of the objects at phase 2. Novel Object preference and Novel Location preference were calculated as time spent on exploration of the novel or relocated object divided by time spent on exploration of both objects. Results from rats that spend less than 3% of their time on exploration of the objects in the baseline condition were excluded from further analysis. Also data from rats scoring 0.00 and 1.00 at Location and Object recognition were removed from further analysis.

### Morris Water Maze (MWM)

The Morris Water Maze consisted in total of six training sessions, two probe trials and three reversed training sessions. The tests were done to assess spatial learning, spatial memory and cognitive flexibility, respectively. The maze entailed a round pool (diameter 140 cm) with an invisible platform, submerged 1-2 cm below water level. The water had a temperature of 26 +/- 1 degrees during the sessions. Visual cues surrounded the pool and divided it into four virtual quadrants. The platform was placed in the target quadrant during the training sessions, removed from the pool during the probe trials and relocated to the opposite quadrant during the reversed training sessions.

The first day consisted of three training sessions. Each training session consisted of three trials for each rat during, which the rats were placed in random order in each quadrant (except the target quadrant). The rats were allowed to find the platform within 60 seconds and left on it for 10 seconds. When the rat did not find the platform within 60 seconds, it was gently guided to the platform and left there for 10 seconds. After the 3 trials the rat was towel dried and placed back in his cage. The period between the training sessions of each rat was at least 2 hours.

The second day started with a probe trial to assess early spatial memory. During the probe trial, the rat was placed in the opposing quadrant of the target quadrant, while the platform was removed. The rat was allowed to swim for 60 seconds and was taken out of the water in the quadrant where the platform was supposed to be. We measured how often the rat crossed the place of the platform and how much time he spent in the target quadrant. After the probe trial the day continued with three more training sessions starting one hour after the probe trial.

At the third day the second probe trial was performed following the same procedure as with probe trial 1. After this probe trial three reversed training sessions were done consisting of three trials each session with the platform placed in the opposing quadrant to assess cognitive flexibility. The order of the rats was different for each day, but not different during the day.

For the MWM the following parameters were calculated: spatial learning: per session the average latency to find the platform to create a learning curve; For the probe trial the number of platform crossings and percentage in target quadrant in the first 15 sec; Memory consolidation as the difference in latency to find the platform in the last learning session and in the first reversal session; Cognitive flexibility obtained from the learning curve in reversal learning.

### Sacrifice and tissue collection

Three days after behavioral testing, rats were anesthetized with pentobarbital (90 mg/kg). The heart was punctured and a blood sample was collected. Blood samples were centrifuged for 10 minutes at 1600 x g, and plasma was collected and stored at -80°C until further analysis. The rat was sacrificed by transcardiac perfusion with saline with Heparin. CSF samples were collected. Half of the brain was divided into the hippocampus, striatum and prefrontal cortex and frozen in liquid nitrogen and stored in -80 for molecular analysis. The other half of the brain was fixated in paraformaldehyde (PFA, 4%) and processed for immunohistochemical analyses.

### Blood samples

Blood samples were taken from all rats equipped with a jugular vein catheter. Blood was sampled 1, 6 and 24 hours (h) after the operation (500 µL). For animals in the chronic and acute J147 treatment group, blood was also collected during the operation (250 µL). Blood samples were centrifuged for 10 minutes at 1600 x g, and plasma was collected and stored at - 80° C until further analysis. Blood samples of the operation were stored at -20°C.

### Plasma levels of J147

To measure actual levels of J147 at the time of surgery, blood samples were collected at the time of surgery. From the jugular vein catheter 250 µl of blood was collected into K3 EDTA coated tubes containing 10 µl of 12.5% Trifluoroacetic Acid (TFA). The tubes were inverted several times to facilitate contact with EDTA and TFA, and subsequently placed on ice until centrifuged. Samples were centrifuged within 30 minutes after collection (4°C, 10 minutes, 1600 x g) to obtain plasma. Collected plasma was stored at -20°C, until further analysis. At all times vials were wrapped in aluminum foil with reference to light sensitivity of J147.

### Brain tissue

Brains were post-fixated in 4% PFA in 0.1M PB for 36-39 hours at room temperature. After the post-fixation brains were washed out from 4% PFA with 0.01 M PB during +/- 96 hours on a shaker, 100 rpm at room temperature. Brains were washed 8 times within the 96 hours. After this, the brains were placed on 30% sucrose in 0.01 M PB overnight at room temperature. When the brains were sunken they were washed with MiliQ water and petted dry. Thereafter the brains were frozen in liquid nitrogen and stored at -80°C until further analysis.

### Immunohistochemical staining

Brains were cut into 25 µm thick sections and stored free floating in 0.01 M PBS + 0.1% Natrium Azide at 4 °C. Before the staining free floating sections were pretreated with 0.3% H202 for 30 min.

To visualize microglia, sections were incubated for 3 days with 1:2500 rabbit-anti IBA-1 in 1% BSA, 0.1% TX at 4 °C. Followed by 2 h incubation with 1:500 goat-anti rabbit secondary antibody at room temperature. All sections were incubated for 1 h with avidin-biotin peroxidase complex (Vectastain ABCkit, Vector, Burlingame, USA) at room temperature. Labeling was visualized using a 0.075 mg/ml DAB solution activated with 0.1% H2O2. All dilutions were made in 0.01 M PBS, except for the DAB which was made in MilliQ.

To visualize newly formed neurons, sections were incubated for 3 days with 1:1000 goat-anti DCX. (Santa Cruz, Dallas, USA) in 3% normal rabbit serum, 0.1% TX at 4 °C, followed by incubation with 1:500 rabbit-anti goat secondary antibody (Jackson, Wet Grove, USA) for 2 h at room temperature. Sections were incubated for 2 h with avidin-biotin peroxidase complex (Vectastain ABCkit, Vector, Burlingame, USA) at room temperature. Labeling was visualized using a 0.075 mg/ml DAB solution activated with 0.1% H₂O₂. All dilutions were made in 0.01 M PBS, except for the DAB which was made in MilliQ.

All sections were thoroughly rinsed with 0.01 M PBS between staining steps. Sections were transferred to glass slides and dehydrated through gradients of ethanol and xylol solutions. Labeling was analyzed blinded to the treatment, in 3 sections per area for each animal. Microglia activation was determined in the Hilus and CA1 region of the hippocampus, the prefrontal cortex (PFC, Zilles's Cg1) and Basolateral Amygdala. Using image analysis software (Image-Pro Plus 6.0), the number of microglia, the average total cell size and average cell body size were determined in an 0.059 square mm area (400 magnification). Since microglia activation was characterized by an increased cell body size and shortening of the dendritic processes (Kreutzberg, 1996), the cell body to total cell size ratio was used as a measure of microglia activation. The number of DCX positive cells was counted in the DG using a light microscope at 40 magnification and corrected for the size of the DG (Van der Borght *et al.,* 2007). The number of DCX positive cells per mm was considered a measure for the number of newly formed neurons in the DG and thus neurogenesis.

### Data Analysis

Experimental groups consists of: 1. non-surgery control food; 2. non-surgery control food +vehicle gavage; 3. surgery control food; 4. surgery control food and vehicle gavage; 5. surgery control food and J147 gavage for acute treatment; 6. surgery and J147 food for chronic treatment. Overall analyses compares group 1 and 2 pooled as non-surgery control; groups 3 and 4 pooled as surgery control to the treated groups 5 and 6.

Results outside twice the standard deviation of its group were regarded as outlier and hence excluded from analysis of comparing means.
Group averages were compared by one way ANOVA (SPSS) followed by post-hoc LSD tests. Data were presented as mean ± SEM. Differences were regarded statistically significant when p=0.05 or smaller. Relevant trends were indicated by p<0.10.

### Results

The study was performed as planned. The behavioral part was studied in 5 cohorts of 12 rats equally divided over the experimental groups. Behavior was recorded and analyzed off line. Tissue and fluids were collected and stored for later processing simultaneously to limit variation.

### Treatment

Plasma samples obtained during surgery in J147-treated rats and 3 control rats were measured for circulating levels of J147. Results of pharmacokinetic measures of both plasma and brain levels of J147 in rats following oral administration of J147 in basis suspension at 20mg/kg and also following intravascular administration at 5mg/kg are shown in Table 2A-C below. When taking the absence of an appropriate peak as zero, mean concentration is 0 ng/ml in controls, 3.103 ng/ml in acutely treated rats and 0.233 ng/ml in chronically treated rats. J147 levels in food were verified as well (467mg/kg). Rats that were fed the J147 diet, gained 32.0±1.8 g during their first week on the diet, which was comparable to 33.5±3.0 g in control fed rats, and suggested no preference or aversion for the diet food. During the first 10 days after surgery and individual housing, rats on the diet ate on average 20±1 g, which was similar to the 20±1 g in surgery with control food and 23±1g in non-surgery controls, verifying the anticipated intake of 10 mg J147/rat/day and further supporting no preference or aversion for the diet food.

### Surgery

All 48 rats that underwent surgery survived and were subjected to the behavioral test. One rat died of unknown cause between behavior testing and sacrifice. Since no clear effect of vehicle gavage was observed, control groups were pooled resulting in the following experimental groups for behavioral analyses: non-surgery control; surgery control; surgery + acute J147; surgery + chronic J147 (n=15 per group).

### Behavior

### Open Field

The open field test was used to examine general exploratory behavior and anxiety. Figure 2 shows no significant effects of either surgery or treatment on time in the center area or distance walked. However notably, surgery rats that were chronically treated with J147 tend to spend more time in the center than the other groups. Actually, these rats did visit the center area significantly more often and showed significantly more rearing behavior.

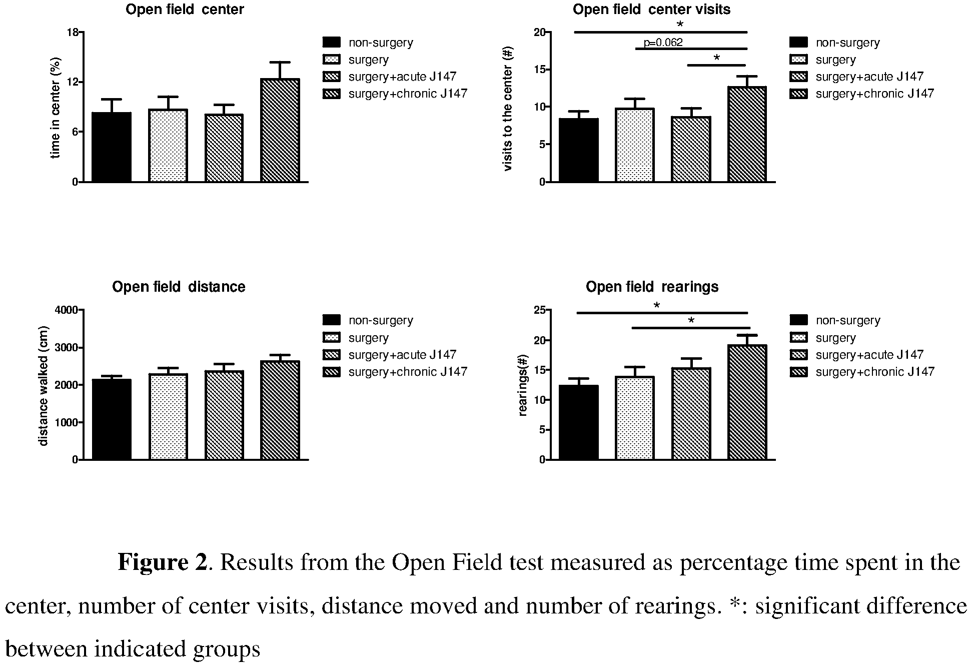

### Novel Object/Novel Location Recognition (NOR/NLR)

Results of the NOR and NLR tests are presented in Table 1. After exploration of the empty cage, rats were presented with 2 similar objects. Overall, groups showed similar interest in the objects, indicating no anxiety towards novelty. Surprisingly, more rats in the control groups (average 45%) had to be excluded because of minor interest in the objects, or interest in only one of the objects, whereas this was less in both treated groups (17%). No significant differences were observed between the groups in preference for the novel or relocated object. The NOR did not discriminate between groups; all groups recognized the novel object (surgery+acute J147; p=0.083). In the NLR test both treated groups performed above random.

**TABLE 1**

| Experimental group | Baseline exploration (% time) | NOR | NLR |
|---|---|---|---|
| Non-surgery | 16.6±3.0 (n=15) | 64±6 (n=9)# | 63±7 (n=7) |
| Control surgery | 19.5±4.5 (n=15) | 69±6 (n=7)# | 60±5 (n=10) |
| Surgerv+acute J147 | 23.6±2.3 (n=15) | 60±5 (n=12) | 61±4 (n=15)# |
| Surgery+chronic J147 | 16.8±2.5 (n=15) | 58±4 (n=12)# | 70±6 (n=11)# |

Table 1. Scores in the Novel Object (NOR) and Novel Location Recognition (NLR) test. #: significantly different from random =50%.

### Morris Water Maze (MWM)

All groups showed a significant learning process, with no differences between groups (Figure 3). Despite the similar spatial learning capacity, spatial memory differed significantly between groups, as can be seen from different times spent in the target quadrant and number of times rats swam over the position of the platform. Memory consolidation and reversal learning were not different between groups. Nevertheless, rats that learned best showed a better memory consolidation, as shown by a highly significant correlation between latency in the last learning session and memory consolidation.

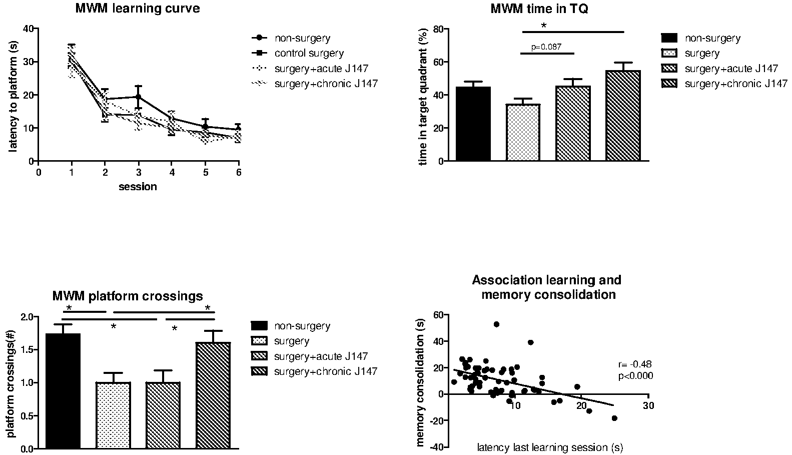

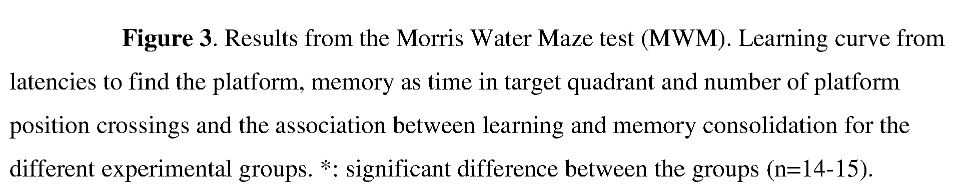

### TABLES 2A-C Pharmacokinetic Measures of Plasma and Brain Levels of J147

Tables 2A-2B. Pharmacokinetic Measures of Plasma and Brain Levels of J147 Formulated in Basis Suspension Administered Orally to Adult Wister Rats at 20mg/kg.

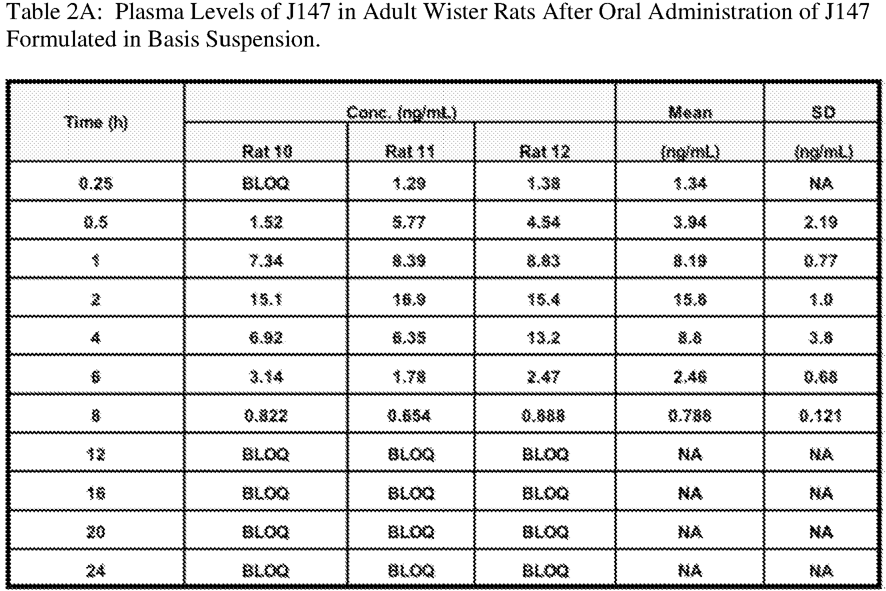

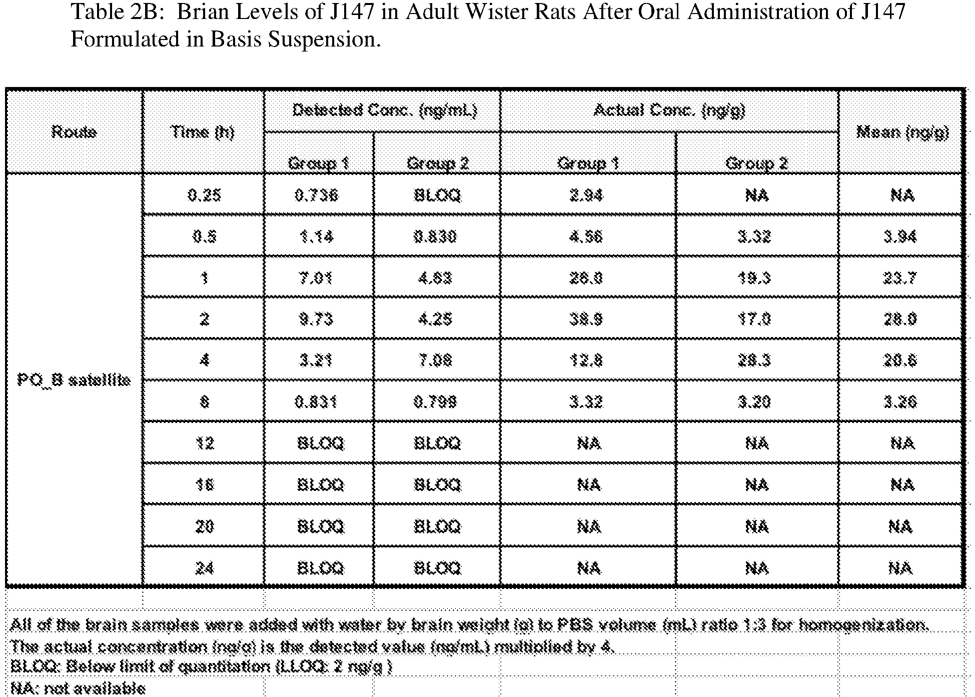

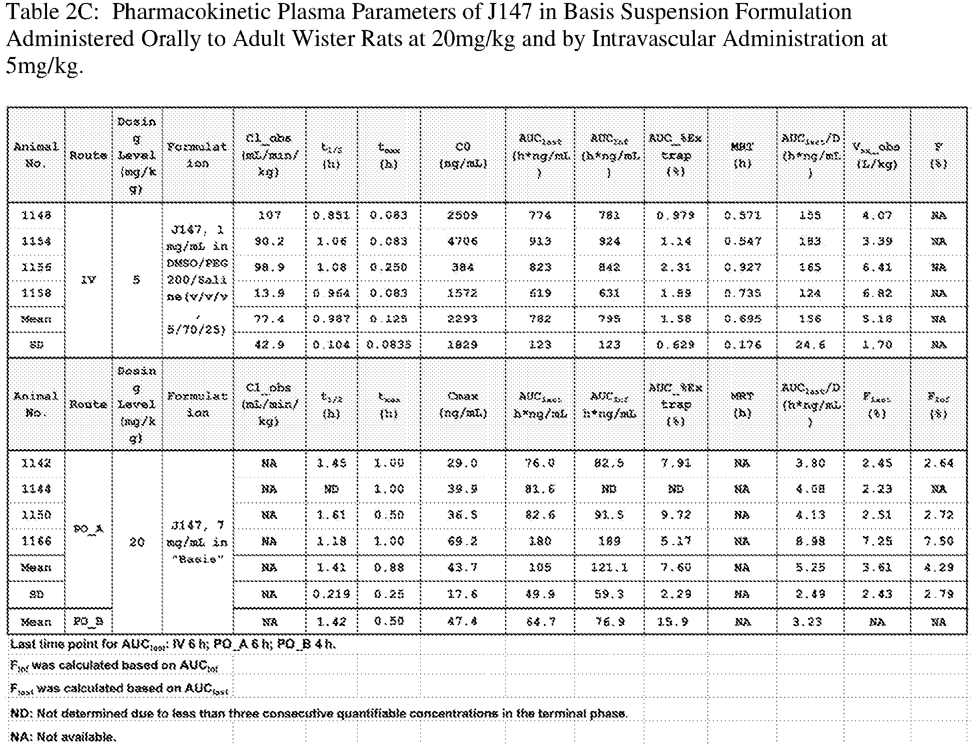

### Conclusions

In general, chronically treated rats showed improvement. These rats lost significantly less body weight after surgery; showed significantly more rearing and visits to the center area in the OF; performed best in NLR and significantly improved spatial memory in the MWM. NOR, spatial and reversal learning were not affected. Data indicate that chronic J174 generally improved conditions after surgery as shown by power body weight loss and behavior in the OF. Moreover, chronic J147 administration was capable of preventing all impaired cognitive aspects after surgery.

### Example 2 - Prophetic Treatment

A male subject, age 60, is scheduled for cardiac bypass surgery. J147 is administered orally at a dose of 1 mg/kg at 24, 12 and 3 hours prior to surgery, with multiple doses administered once a day starting on the day after surgery and extending for a time period of 1 to 6 weeks after surgery. The subject is assessed for POCD by administering one or more suitable cognitive tests on the day before, and 1 week after surgery. One week after surgery the subject is determined to have no loss of cognitive function.

Citation of any patent, patent application, publication or any other document is not an admission that any of the foregoing is pertinent prior art, nor does it constitute any admission as to the contents or date of these publications or documents.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described herein.

All of the features disclosed herein may be combined in any combination. Each feature disclosed in the specification may be replaced by an alternative feature serving a same, equivalent, or similar purpose. Thus, unless expressly stated otherwise, disclosed features (*e.g*., antibodies) are an example of a genus of equivalent or similar features.

As used herein, all numerical values or numerical ranges include integers within such ranges and fractions of the values or the integers within ranges unless the context clearly indicates otherwise. Further, when a listing of values is described herein (*e.g*., about 50%, 60%, 70%, 80%, 85% or 86%) the listing includes all intermediate and fractional values thereof *(e.g.,* 54%, 85.4%). Thus, to illustrate, reference to 80% or more identity, includes 81%, 82%, 83%, 84%, 85%, 86%, 87%, 88%, 89%, 90%, 91%, 92%, 93%, 94% etc., as well as 81.1%, 81.2%, 81.3%, 81.4%, 81.5%, etc., 82.1%, 82.2%, 82.3%, 82.4%, 82.5%, etc., and so forth.

Reference to an integer with more (greater) or less than includes any number greater or less than the reference number, respectively. Thus, for example, a reference to less than 100, includes 99, 98, 97, etc. all the way down to the number one (1); and less than 10, includes 9, 8, 7, etc. all the way down to the number one (1).

As used herein, all numerical values or ranges include fractions of the values and integers within such ranges and fractions of the integers within such ranges unless the context clearly indicates otherwise. Thus, to illustrate, reference to a numerical range, such as 1-10 includes 1, 2, 3, 4, 5, 6, 7, 8, 9, 10, as well as 1.1, 1.2, 1.3, 1.4, 1.5, etc., and so forth. Reference to a range of 1-50 therefore includes 1, 2, 3, 4, 5, 6, 7, 8, 9, 10, 11, 12, 13, 14, 15, 16, 17, 18, 19, 20, etc., up to and including 50, as well as 1.1, 1.2, 1.3, 1.4, 1.5, etc., 2.1, 2.2, 2.3, 2.4, 2.5, etc., and so forth.

Reference to a series of ranges includes ranges which combine the values of the boundaries of different ranges within the series. Thus, to illustrate reference to a series of ranges, for example, of 1-10, 10-20, 20-30, 30-40, 40-50, 50-60, 60-75, 75-100, 100-150, 150-200, 200-250, 250-300, 300-400, 400-500, 500-750, 750-1,000, 1,000-1,500, 1,500-2,000, 2,000-2,500, 2,500-3,000, 3,000-3,500, 3,500-4,000, 4,000-4,500, 4,500-5,000, 5,500-6,000, 6,000-7,000, 7,000-8,000, or 8,000-9,000, includes ranges of 10-50, 50-100, 100-1,000, 1,000-3,000, 2,000-4,000, etc.

The invention is generally disclosed herein using affirmative language to describe the numerous embodiments and aspects. The invention also specifically includes embodiments in which particular subject matter is excluded, in full or in part, such as substances or materials, method steps and conditions, protocols, or procedures. For example, in certain embodiments or aspects of the invention, materials and/or method steps are excluded. Thus, even though the invention is generally not expressed herein in terms of what the invention does not include aspects that are not expressly excluded in the invention are nevertheless disclosed herein.

Some embodiments of the technology described herein suitably can be practiced in the absence of an element not specifically disclosed herein. Accordingly, in some embodiments the term "comprising" or "comprises" can be replaced with "consisting essentially of" or "consisting of" or grammatical variations thereof. The term "a" or "an" can refer to one of or a plurality of the elements it modifies (*e.g.*, "a reagent" can mean one or more reagents) unless it is contextually clear either one of the elements or more than one of the elements is described. The term "about" as used herein refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a weight of "about 100 grams" can include weights between 90 grams and 110 grams. The term, "substantially" as used herein refers to a value modifier meaning "at least 95%", "at least 96%","at least 97%","at least 98%", or "at least 99%" and may include 100%. For example, a composition that is substantially free of X, may include less than 5%, less than 4%, less than 3%, less than 2%, or less than 1% of X, and/or X may be absent or undetectable in the composition.

## Claims

1. A compound of Formula IV; 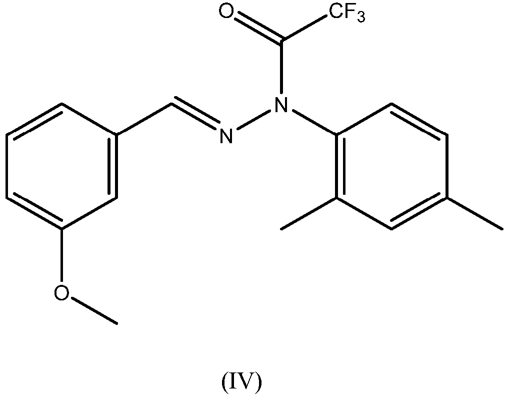
or a pharmaceutically acceptable salt, stereoisomer or tautomer thereof,
for use in preventing, reducing, delaying or treating post-operative cognitive dysfunction (POCD), or one or more symptoms thereof, in a subject, wherein the POCD is induced by, or is a result of a medical procedure.

2. The compound for use according to claim 1, wherein the medical procedure comprises major surgery.

3. The compound for use according to claim 2, wherein the major surgery comprises a surgical procedure having a duration of 0.5 hour to 20 hours.

4. The compound for use according to any one of claims 1 to 3, wherein the medical procedure is selected from cardiac surgery, angioplasty, organ transplant surgery, complete or partial removal of an organ or tissue, brain surgery, bone replacement or repair surgery, abdominal surgery, facial reconstruction or repair surgery, and pelvic floor surgery.

5. The compound for use according to any one of claims 1 to 4, wherein the subject is over the age of 60, or over the age of 65.

6. The compound for use according to any one of claims 1 to 5, wherein the subject is human.

7. The compound for use according to any one of claims 1 to 6, wherein the subject displays stable cognitive function prior to the medical procedure or wherein the subject was not previously diagnosed with a cognitive disorder or neurodegenerative disease prior to the medical procedure.

8. The compound for use according to any one of claims 1 to 6, wherein the subject is diagnosed with a neurodegenerative disease prior to the medical procedure.

9. The compound for use according to claim 8, wherein the neurodegenerative disease is selected from Alzheimer's disease, Parkinson's disease, Huntington's disease, amyotrophic lateral sclerosis, glaucoma, retinal degeneration, macular degeneration, age-related hearing loss, mild cognitive impairment, dementia, progressive supranuclear palsy, spinocerebellar ataxia, retinal neuropathy, peripheral neuropathy, diabetic neuropathy, background neuropathy, familial amyloid polyneuropathy, systemic senile amyloidosis, prion disease, scrapie, bovine spongiform encephalopathy, Creutzfeldt-Jakob disease, Gerstmann-Straussler-Scheinker syndrome and amyloidosis.

10. The compound for use according to any one of claims 1 to 9, wherein the compound is administered prior to, perioperatively, during or after the medical procedure or surgery.

11. The compound for use according to any one of claims 1 to 10, wherein the compound is administered at a dose of 0.5 mg/kg to 50 mg/kg.

12. The compound for use according to any one of claims 1 to 11, wherein the compound is administered orally or intravenously.

## Patentansprüche

1. Eine Verbindung der Formel IV: 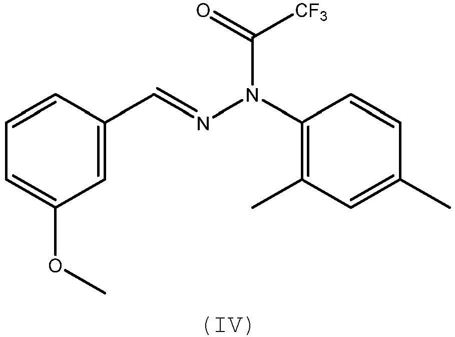 oder ein pharmazeutisch verträgliches Salz, Stereoisomer oder Tautomer davon zur Verwendung zum Verhüten, Verringern, Verzögern oder Behandeln einer postoperativen kognitiven Dysfunktion (POCD) oder eines oder mehrerer Symptome davon bei einem Subjekt, wobei die POCD durch einen medizinischen Eingriff verursacht wird oder eine Folge davon ist.

2. Die Verbindung zur Verwendung nach Anspruch 1, wobei der medizinische Eingriff eine große Operation umfasst.

3. Die Verbindung zur Verwendung nach Anspruch 2, wobei die große Operation einen chirurgischen Eingriff mit einer Dauer von 0,5 Stunden bis 20 Stunden umfasst.

4. Die Verbindung zur Verwendung nach einem der Ansprüche 1 bis 3, wobei der medizinische Eingriff aus der Herzchirurgie, Angioplastie, Organtransplantationschirurgie, vollständigen oder teilweisen Entfernung eines Organs oder Gewebes, Hirnchirurgie, Knochenersatz- oder Knochenwiederherstellungschirurgie, Abdomenchirurgie, Gesichtsrekonstruktions- oder Gesichtswiederherstellungschirurgie und Beckenbodenchirurgie ausgewählt ist.

5. Die Verbindung zur Verwendung nach einem der Ansprüche 1 bis 4, wobei das Subjekt mehr als 60 oder mehr als 65 Jahre alt ist.

6. Die Verbindung zur Verwendung nach einem der Ansprüche 1 bis 5, wobei das Subjekt ein Mensch ist.

7. Die Verbindung zur Verwendung nach einem der Ansprüche 1 bis 6, wobei das Subjekt vor dem medizinischen Eingriff eine stabile kognitive Funktion aufweist oder wobei bei dem Subjekt vor dem medizinischen Eingriff keine kognitive Störung oder neurodegenerative Erkrankung diagnostiziert wurde.

8. Die Verbindung zur Verwendung nach einem der Ansprüche 1 bis 6, wobei bei dem Subjekt vor dem medizinischen Eingriff eine neurodegenerative Erkrankung diagnostiziert wurde.

9. Die Verbindung zur Verwendung nach Anspruch 8, wobei die neurodegenerative Erkrankung aus Alzheimer-Erkrankung, Parkinson-Erkrankung, Chorea Huntington, amyotropher Lateralsklerose, Glaukom, Netzhautdegeneration, Makuladegeneration, altersbedingter Schwerhörigkeit, schwacher kognitiver Beeinträchtigung, Demenz, progressiver supranukleärer Blickparese, spinozerebellärer Ataxie, retinaler Neuropathie, peripherer Neuropathie, Diabetesneuropathie, Hintergrundneuropathie, familiärer Amyloidpolyneuropathie, systemischer seniler Amyloidose, Prionenerkrankung, Scrapie, boviner spongiformer Enzephalopathie, Creutzfeld-Jakob-Krankheit, Gerstmann-Sträussler-Scheinker-Syndrom und Amyloidose ausgewählt ist.

10. Die Verbindung zur Verwendung nach einem der Ansprüche 1 bis 9, wobei die Verbindung vor, perioperativ, während oder nach dem medizinischen Eingriff oder der Operation verabreicht wird.

11. Die Verbindung zur Verwendung nach einem der Ansprüche 1 bis 10, wobei die Verbindung mit einer Dosis von 0,5 mg/kg bis 50 mg/kg verabreicht wird.

12. Die Verbindung zur Verwendung nach einem der Ansprüche 1 bis 11, wobei die Verbindung oral oder intravenös verabreicht wird.

## Revendications

1. Composé de Formule IV ; 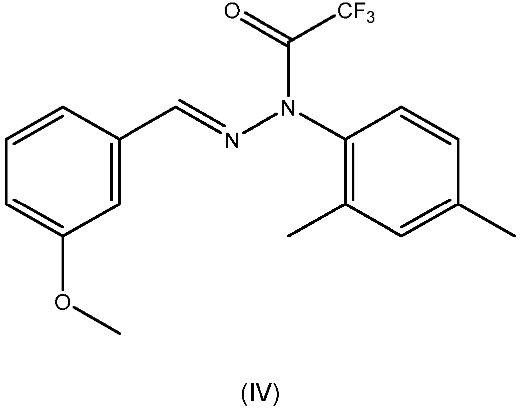 ou sel pharmaceutiquement acceptable, stéréoisomère ou tautomère de celui-ci, à utiliser dans la prévention, la réduction, le retardement ou le traitement du dysfonctionnement cognitif post-opératoire (POCD), ou d'un ou plusieurs des symptômes de celui-ci, chez un sujet, dans lequel le POCD est induit par ou résulte d'une procédure médicale.

2. Composé à utiliser selon la revendication 1, dans lequel la procédure médicale comprend une intervention chirurgicale majeure.

3. Composé à utiliser selon la revendication 2, dans lequel l'intervention chirurgicale majeure comprend une procédure chirurgicale d'une durée de 0,5 heure à 20 heures.

4. Composé à utiliser selon l'une quelconque des revendications 1 à 3, dans lequel la procédure médicale est choisie parmi la chirurgie cardiaque, l'angioplastie, la chirurgie de transplantation d'organe, l'ablation complète ou partielle d'un organe ou d'un tissu, la chirurgie cérébrale, la chirurgie de remplacement ou de réparation osseuse, la chirurgie abdominale, la chirurgie de reconstruction ou de réparation faciale, et la chirurgie du plancher pelvien.

5. Composé à utiliser selon l'une quelconque des revendications 1 à 4, dans lequel le sujet est âgé de plus de 60 ans ou de plus de 65 ans.

6. Composé à utiliser selon l'une quelconque des revendications 1 à 5, dans lequel le sujet est humain.

7. Composé à utiliser selon l'une quelconque des revendications 1 à 6, dans lequel le sujet présente une fonction cognitive stable avant la procédure médicale ou dans lequel le sujet n'a pas été diagnostiqué avec un trouble cognitif ou une maladie neurodégénérative avant la procédure médicale.

8. Composé à utiliser selon l'une quelconque des revendications 1 à 6, dans lequel une maladie neurodégénérative a été diagnostiquée chez le sujet avant la procédure médicale.

9. Composé à utiliser selon la revendication 8, dans lequel la maladie neurodégénérative est choisie parmi la maladie d'Alzheimer, la maladie de Parkinson, la maladie de Huntington, la sclérose latérale amyotrophique, le glaucome, la dégénérescence rétinienne, la dégénérescence maculaire, la perte auditive liée à l'âge, la déficience cognitive légère, la démence, la paralysie supranucléaire progressive, l'ataxie spinocérébelleuse, la neuropathie rétinienne, la neuropathie périphérique, la neuropathie diabétique, la neuropathie de fond, la polyneuropathie amyloïde familiale, l'amylose sénile systémique, la maladie à prions, la tremblante du mouton, l'encéphalopathie spongiforme bovine, la maladie de Creutzfeldt-Jakob, le syndrome de Gerstmann-Straussler-Scheinker et l'amyloïdose.

10. Composé à utiliser selon l'une quelconque des revendications 1 à 9, dans lequel le composé est administré avant, en période periopératoire, pendant ou après la procédure médicale ou l'intervention chirurgicale.

11. Composé à utiliser selon l'une quelconque des revendications 1 à 10, dans lequel le composé est administré à une dose de 0,5 mg/kg à 50 mg/kg.

12. Composé à utiliser selon l'une quelconque des revendications 1 à 11, dans lequel le composé est administré par voie orale ou intraveineuse.
